# EUROPEAN PATENT APPLICATION

(11) **EP 2 989 973 A1**
(43) Date of publication of application: **02.03.2016**
(21) Application number: 15182556.9
(22) Date of filing: 26.08.2015
(51) Int. Cl.: A61B 5/00, A61B 5/11, A61B 5/01, A61B 5/053

(54) **ELECTRONIC DEVICE AND SLEEP MONITORING METHOD IN ELECTRONIC DEVICE**

(30) Priority: 26.08.2014 KR 20140111676
(71) Applicant: Samsung Electronics Co., Ltd., Suwon-si, Gyeonggi-do 443-742 (KR)
(72) Inventor: CHOI, Byung-Hun, Suwon-si, Gyeonggi-do 443-742 (KR); KIM, Do-Yoon, Suwon-si, Gyeonggi-do 443-742 (KR); CHO, Jae-Geol, Suwon-si, Gyeonggi-do 443-742 (KR)
(74) Representative: Grünecker Patent- und Rechtsanwälte PartG mbB

(57) **Abstract**

Wearable electronic devices, systems, and methods for monitoring sleep are described. In one method, motion sensor values of the current motion of the electronic device are acquired and the change in motion intensity of an electronic device is calculated by comparing motion sensor values over two or more time periods. If the change in motion intensity fits a predetermined pattern, it is determined whether the electronic device is currently being worn. If it is determined that the electronic device is currently being worn, sleep monitoring is performed.

## Description

### BACKGROUND

### 1. Field of the Disclosure

The present invention relates generally to an electronic device and, more particularly, to a sleep monitoring method in an electronic device.

### 2. Description of the Related Art

Owing to recent remarkable developments in information, communication, and semiconductor techniques, the distribution, use, and capabilities of electronic devices have rapidly increased. In particular, recent electronic devices have gotten to the stage of mobile convergence where electronic devices have embraced many capabilities beyond their original primary purpose. Typically, a mobile communication terminal is capable of various functions, such as watching TV *(e.g.,* mobile broadcasting, such as Digital Multimedia Broadcasting (DMB) or Digital Video Broadcasting (DVB)), playing music *(e.g.,* MP3 (Motion Picture Experts Group Audio Layer-3)), taking and viewing photographs and videos, and connecting to the Internet, in addition to its general telecommunication function, such as voice call or message transmission/reception.

Recently, a different kind of electronic device, a wearable electronic device, has been developed, which may take the form of, for example, a watch, a headset, or glasses which are worn on a part of the user's body. Wearable electronic devices may be operated independently, or interlocked with another electronic device, for example, a mobile communication terminal, to be operated as a companion device so that the wearable electronic device can provide at least some of the functions of the mobile communication terminal (e.g., voice call, message, and streaming) to the user.

Also recently, according to the changing currents of the times, as the quality of life has improved with the increase of income, and the birthrate has decreased as the aging population has increased, the healthcare paradigm has changed from how to treat diseases to how to control and prevent diseases. Accordingly, research and development concerning chronic disease and healthcare management services have been and are being actively carried out. As part of this, sleep has received as much attention as diet or exercise in terms of being a health issue, and related industries have grown rapidly. Techniques and electronic devices for monitoring a user's sleep state have been and are currently being developed.

However, the conventional method used in electronic devices that monitor sleep typically requires user input as part of the monitoring function. Thus, for example, the user has to record a sleep start time and a sleep finish time using, e.g., a button or other input method in order for the device to continuously measure data for sleep monitoring. The reliance on active user input of conventional methods is problematic for a number of reasons, including the questionable accuracy of the resulting monitoring data.

Therefore, there is need for apparatuses (including, but not limited to, wearable electronic devices), systems, and methods for monitoring sleep without requiring the user to actively provide input.

### SUMMARY

The present invention has been made to address at least the problems and disadvantages described above and to provide at least the advantages described below. In one aspect of the present invention, an electronic device and method for sleep monitoring are provided, in accordance with which sleep is automatically monitored, such that the sleep start time and sleep finish time are automatically detected without user input.

According to another aspect of the present invention, an electronic device capable of automatic sleep monitoring is provided, by which sleep is automatically monitored based on the detection of motion by the electronic device while worn by the user.

According to yet another aspect of the present invention, an electronic device and method for sleep monitoring are provided, in which sleep is prevented from being erroneously recognized when the user does not wear the electronic device.

According to still another aspect of the present invention, an electronic device and method for sleep monitoring are provided, in which a sensor for sleep monitoring is activated when it is determined that the user is currently wearing the electronic device so that power consumption is reduced as compared to a case in which the sensor for sleep monitoring is periodically activated.

According to one aspect of the present invention, an electronic device includes a motion sensor that senses a motion of the electronic device; a biometric sensor that senses a biometric signal; and a processor that calculates a change in motion intensity of the electronic device by comparing output values of the motion sensor over two or more time periods, activates the biometric sensor to determine whether the electronic device is currently being worn when the change in motion intensity over two or more time periods corresponds to a predetermined pattern, and controls the electronic device to perform sleep monitoring when it is determined that the electronic device is currently being worn.

According to another aspect of the present invention, a method for sleep monitoring by an electronic device is provided, including acquiring motion sensor values according to a current motion of the electronic device; calculating a change in motion intensity of the electronic device by comparing the motion sensor values over two or more time periods; determining whether the change in motion intensity over two or more time periods corresponds to a predetermined pattern; if it is determined that the change in motion intensity corresponds to the predetermined pattern, determining whether the electronic device is currently being worn; and, if it is determined that the electronic device is currently being worn, performing sleep monitoring.

According to yet another aspect of the present invention, a method for sleep monitoring in an electronic device includes acquiring a motion sensor value according to a current motion of the electronic device; transmitting the acquired motion sensor value to an external electronic device; if a sensor module activation request is received from the external electronic device, activating a sensor module; acquiring a sensor value using the activated sensor module; transmitting the sensor value to the external electronic device for determining whether the electronic device is currently being worn; receiving a sleep monitoring mode request from the external electronic device when the external electronic device determines, based on the transmitted sensor value, that the electronic device is currently being worn; and performing sleep monitoring according to the received sleep monitoring mode request by detecting and transmitting sensor values to the external electronic device.

According to still another aspect of the present invention, a method for sleep monitoring in an electronic device includes receiving motion sensor values from the electronic device, the motion sensor values detected according to a current motion of the electronic device; calculating a change in motion intensity of the electronic device by comparing the motion sensor values over two or more time periods and determining whether the change in motion intensity over two or more time periods corresponds to a predetermined pattern; if it is determined that the change in motion intensity between two or more sections corresponds to the predetermined pattern, transmitting a sensor module activation request to the electronic device; receiving a sensor value from the electronic device; determining whether the electronic device is currently being worn using the received sensor value; if it is determined that the electronic device is currently being worn, transmitting a sleep monitoring mode request to the electronic device; and performing sleep monitoring by using sensor values received from the electronic device while it is in sleep monitoring mode.

According to still yet another aspect of the present invention, a non-transitory computer-readable storage medium is provided that stores a sleep monitoring program, wherein the program executes, in an electronic device, the operations of acquiring motion sensor values according to a current motion of the electronic device; calculating a change in motion intensity of the electronic device by comparing the motion sensor values over two or more time periods; determining whether the change in motion intensity over two or more time periods corresponds to a predetermined pattern; if the change in motion intensity corresponds to the predetermined pattern, determining whether the electronic device is currently being worn by activating a biometric sensor; and, if it is determined that the electronic device is currently being worn, performing sleep monitoring.

### BRIEF DESCRIPTION OF THE DRAWINGS

The above and other aspects, features, and advantages of certain embodiments of the present invention will become more apparent from the following detailed description taken in conjunction with the accompanying drawings, in which:
FIG 1 shows a user with a wearable electronic device for sleep monitoring according to an embodiment of the present invention;
FIG 2 illustrates a network environment including an electronic device according to an embodiment of the present invention;
FIG 3 is a block diagram of a sensor module according to an embodiment of the present invention;
FIGS. 4 to 7 are views illustrating examples of electronic devices being worn by a user according to various embodiments of the present invention;
FIG. 8 is a perspective view for describing a worn state of an electronic device according to an embodiment of the present invention;
FIG. 9A is a perspective view of the front of the main body of an electronic device according to an embodiment of the present invention;
FIG. 9B is a perspective view of the rear of the main body of the electronic device according to an embodiment of the present invention;
FIG 10 has an expanded view of a sensor module on the rear of the main body of the electronic device according to an embodiment of the present invention;
FIG 11 is a flowchart of a method of sleep monitoring in an electronic device according to an embodiment of the present invention;
FIG 12 is a table of change patterns in motion intensity, according to a first embodiment;
FIG 13 is a flowchart of a method of sleep monitoring according to the first embodiment;
FIG 14 is a table of change patterns in motion intensity over three time periodssection, according to the second embodiment;
FIGS. 15A and 15B comprise a flowchart of a method of sleep monitoring according to change patterns in motion intensity over three time periods, according to the second embodiment;
FIG 16 is a flowchart of a method of determining whether the electronic device is currently being worn using a photo sensor according to an embodiment of the present invention;
FIG. 17 is a flowchart of a method of determining whether the electronic device is currently being worn using a GSR sensor according to an embodiment of the present invention;
FIG. 18 is a flowchart of a method of determining whether the electronic device is currently being worn using a temperature sensor according to an embodiment of the present invention;
FIG. 19 is a graph of motion intensity over time according to an embodiment of the present invention;
FIG. 20 is a graph showing the result of determining whether the electronic device is worn according to an embodiment of the present invention;
FIGS. 21A, 21B, and 21C illustrate examples of sleep monitoring screens of the electronic device according to an embodiment of the present invention;
FIG. 22 is a view illustrating an electronic device and an external electronic device according to an embodiment of the present invention;
FIG. 23 is a flowchart of sleep monitoring operations using the electronic device and the external electronic device according to an embodiment of the present invention; and
FIG 24 is a block diagram of an electronic device according to an embodiment of the present invention.

### DETAILED DESCRIPTION OF EMBODIMENTS OF THE PRESENT INVENTION

Embodiments of the present invention are described below in detail with reference to the accompanying drawings. However, the description of these embodiments is not intended to limit the present invention, but rather to illustrate examples of the present invention. It shall be appreciated that all the possible modifications, changes, equivalents, and substitutions of the various embodiments are also included within the technical scope of the present invention. In the drawings, identical or similar reference numerals may be used to designate identical or similar elements.

The terms "include" or "may include" refer to the existence of a corresponding disclosed function, operation or component which can be used in various embodiments of the present invention but does not limit the present invention in any way. In the present disclosure, terms such as "include" or "have" may be construed to denote a certain characteristic, number, step, operation, constituent element, component or a combination thereof, but may not be construed to exclude the existence of or a possibility of one or more other characteristics, numbers, steps, operations, constituent elements, components or combinations thereof.

In the present disclosure, the expressions "or" or "at least one of A or/and B" indicates any and all combinations of the terms listed together. For example, the expression "at least A or/and B" includes A, B, and both A and B.

Expressions such as "first", "second", or the like used in the present disclosure are used merely for convenience of reference and explanation to distinguish various components and/or elements, and, unless expressly stated, do not limit the sequence and/or importance of the components/elements. Thus, for example, a first component/element may be named a second component/element or the second component/element also may be named the first component element without departing from the scope of the present disclosure,.

If one component/element is described as "coupled" or "connected" to another component/element, the first component/element may be directly coupled or connected to the second component/element, or a third component/element may be "coupled" or "connected" between the first and second component/elements. Conversely, when one component/element is "directly coupled" or "directly connected' to another component/element, it may be construed that a third component/element does not exist between the first component/element and the second component/element.

As used herein, the singular forms of terms are intended to include the plural forms as well, unless the context clearly indicates otherwise.

Unless defined differently, all terms used herein, which include technical or scientific terms, have the same meaning as would be understood by a person of ordinary skill in the art to which the present disclosure belongs. Such terms as those defined in a generally used dictionary are to be interpreted to have meanings commensurate with the contextual meaning of those terms in the relevant field of the art, and are not to be interpreted to have ideal or excessively formal meanings, except those clearly defined so in the present disclosure.

According to various embodiments of the present invention, sleep is automatically monitored without requiring user input. For example, in some embodiments, the sleep start time and the sleep finish time are automatically detected even if the user does not input the sleep start time and the sleep finish time.

According to various embodiments of the present invention, sleep is automatically monitored based on the detection of motion of an electronic device worn by the user.

According to various embodiments of the present invention, when sleep is monitored based on the detection of motion of the electronic device, sleep is not recognized when the user does not wear the electronic device.

According to various embodiments of the present invention, the sleep monitoring sensor is activated by the motion of the wearable electronic device, for example, when a pattern of motion intensity of the electronic device worn by the user corresponds to a predetermined pattern. Thus, power consumption can be reduced as compared to a case in which the sleep monitoring sensor is periodically activated.

An electronic device capable of sleep monitoring according to embodiments of the present invention may be, for example, a smart phone, a tablet personal computer (PC), a mobile phone, a video phone, an e-book reader, a desktop PC, a laptop PC, a netbook computer, a personal digital assistant (PDA), a portable multimedia player (PMP), an MP3 player, a mobile medical device, a camera, or a wearable electronic device, such as,e.g., a head-mounted-device (HMD), electronic glasses, electronic clothes, an electronic bracelet, an electronic necklace, an electronic accessory, an electronic tattoo, or a smart watch.

According to some embodiments of the present invention, the electronic device may be a smart home appliance with a camera function, such as, for example, a television, a Digital Video Disk (DVD) player, an audio system, a refrigerator, an air conditioner, a vacuum cleaner, an oven, a microwave oven, a washing machine, an air cleaner, a set-top box, a TV box (e.g., Samsung HomeSync™, Apple TV™, or Google TV™), a game console, an electronic dictionary, an electronic key, a camcorder, or an electronic picture frame.

According to some embodiments of the present invention, the electronic device may a medical appliance (e.g. Magnetic Resonance Angiography (MRA), Magnetic Resonance Imaging (MRI), Computed Tomography (CT) machine, or an ultrasonic machine), a navigation device, a Global Positioning System (GPS) receiver, an Event Data Recorder (EDR), a Flight Data Recorder (FDR), an automotive infortainment device, naval electronic equipment (e.g. navigation equipments for ships, gyrocompasses, or the like), an avionic device, a security device, a HMD for a vehicle, an industrial or home robot, an Automatic Teller Machine (ATM), or a Point Of Sale (POS) device.

According to some embodiments of the present invention, the electronic device may be furniture or a part of a building/structure, an electronic board, an electronic signature receiving device, a projector, or a type of measuring device (for example, a water meter, an electric meter, a gas meter, a radio wave meter, and the like) including a camera function.

An electronic device according to various embodiments of the present invention may be a combination of one or more of above described various devices. Also, an electronic device according to various embodiments of the present invention may be a flexible device. An electronic device according to various embodiments of the present invention is not limited to the above described devices.

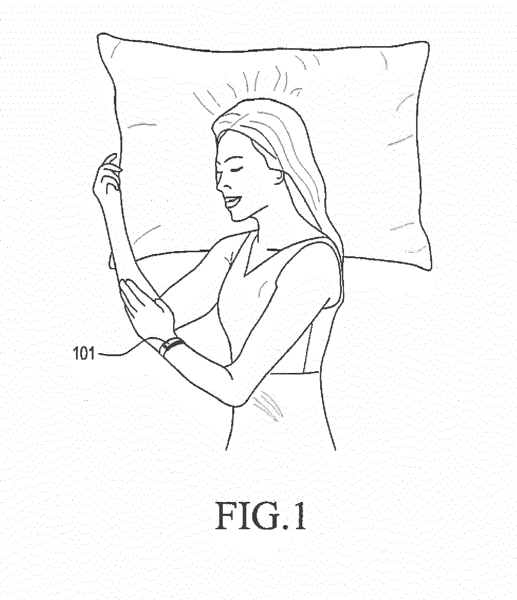

FIG. 1 shows a user wearing an electronic device for sleep monitoring according to an embodiment of the present invention. Referring to FIG. 1, when electronic device 101 is worn by the user, it can monitor the user's sleep state. The electronic device 101 may be worn on any suitable portion of the user's body, including, for example, the wrist, arm, head, or ankle.

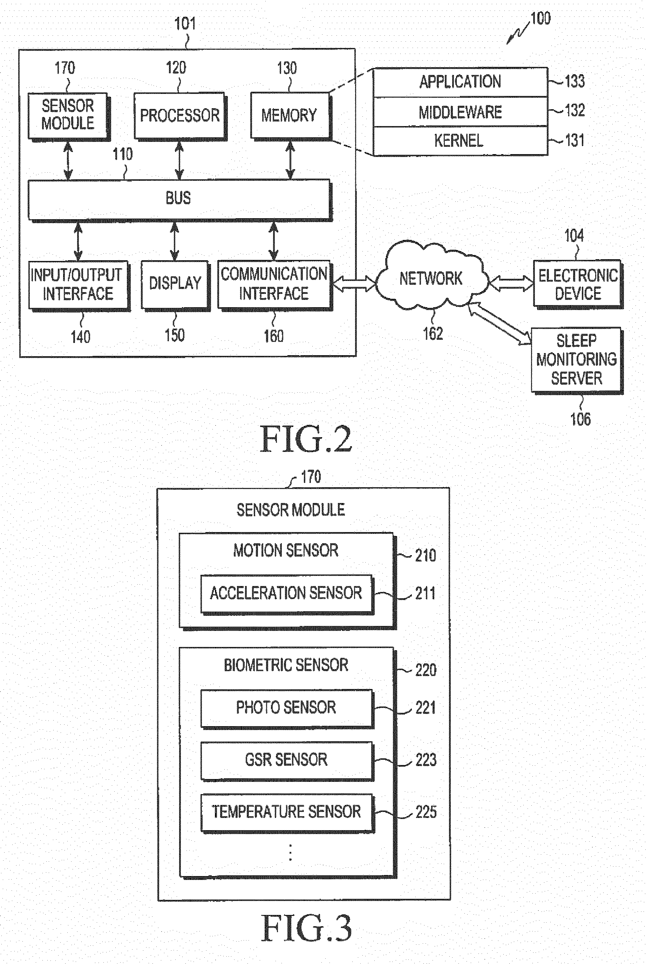

FIG. 2 illustrates a network environment 100 including the electronic device 101, a network 162, another electronic device 104, and a sleep monitoring server 106, according to various embodiments of the present invention. Electronic device 101 in FIG. 2 includes a bus 110, a processor 120, a memory 130, an input/output interface 140, a display 150, a communication interface 160, and a sensor module 170.

The bus 110 is a circuit that interconnects the above-described components with each other, and delivers communication signals (e.g., control messages) between the above-described components.

The processor 120 receives, e.g., data, messages, and commands from the other components described above (*e.g*., the memory 130, the input/output interface 140, the display 150, the communication interface 160, or the sensor module 170) through, for example, the bus 110. If the processor receives a command, it decodes the received command, and executes an arithmetic operation or data processing according to the decoded command. According to one embodiment, the processor 120 calculates the change in motion intensity of the electronic device 101 over a predetermined time unit using a motion sensor. When a detected change in motion intensity fits a predetermined pattern, the processor 120 determines whether the electronic device 101 is being currently worn by the user using a biometric sensor. If the electronic device 101 is currently being worn, the processor 120 performs sleep monitoring. According to one embodiment, if it is determined that the electronic device 101 is not currently being worn, one or more components of electronic device 101 enter a power saving mode.

The memory 130 stores, *e.g.,* instructions and/or data, including instructions/data received from, or generated by, the processor 120 or any other components (*e.g*., the input/output interface 140, the display 150, the communication interface 160, or the sensor module 170). The memory 130 includes programming modules, such as kernel 131, middleware 132, and one or more applications 133. Each of the programming modules may be configured by software, firmware, hardware, or a combination of at least two thereof.

The kernel 131 may control or manage system resources (e.g., the bus 110, the processor 120, and the memory 130) used for executing operations or functions implemented in the other programming modules, for example, the middleware 132 or the applications 133. In addition, the kernel 131 may provide an interface that allows the middleware 132 or the applications 133 to access individual components of the electronic device 101 so as to control or manage the individual components of the electronic device 101.

The middleware 132 may act as an intermediary to allow the applications 133 to communicate with the kernel 131 so as to exchange data. In addition, the middleware 132 may execute a control for a task request (*e.g*., scheduling or load balancing), for example, using a method of allocating a priority capable of using a system resource of the electronic device 101 (*e.g*., the bus 110, the processor 120, or the memory 130) to, for example, at least one application among the applications 133 in connection with task requests received from the applications 133. According to various embodiments of the present invention, the applications 133 may include a Short Message Service (SMS)/Multimedia Message Service (MMS) application, an e-mail application, a calendar application, an alarm application, a health care application (e.g., an application for monitoring a sleep state application or an application for measuring, for example, the user's motion or blood sugar), or an environment information application (e.g., an application for providing atmospheric pressure, humidity, or temperature information). Additionally or alternatively, the applications 133 may be applications related to information exchange between the electronic device 101 and an external electronic device *(e.g.,* electronic device 104). Information exchange applications include, for example, a notification relay application for relaying specific information to the external electronic device and/or a device management application for managing the external electronic device.

A notification relay application may include, ror example, a function for relaying notification information from other applications (such as, *e.g.,* a sleep monitoring application, SMS/MMS application, e-mail application, health care application, or environment information application) to an external electronic device (e.g., the electronic device 104). Additionally or alternatively, the notification relay application may receive notification information from, for example, the external electronic device *(e.g.,* the electronic device 104) and provide the notification information to a user.

The device management application manages (e.g., installs, deletes, or updates), for example, at least some functions of an external electronic device *(e.g.,* the electronic device 104) that communicates with the electronic device 101 (functions such as, *e.g.,* turning ON/OFF the electronic device itself or some components thereof or adjusting the brightness or resolution of a display), applications operated in the external device, and/or services (*e.g.*, a call service or a message service) provided from the external device.

According to various embodiments, the applications 133 may include an application designated according to the functionality or an attribute (e.g., the kind of electronic device) of the external electronic device. For example, when the external electronic device is an MP3 player, applications 133 may include an application related to music reproduction. Similarly, when the external electronic device is a mobile medical device, applications 133 may include an application related to healthcare. Similarly, when the external electronic device is capable of performing sleep monitoring, applications 133 may include an application related to the sleep monitoring. According to one embodiment, applications 133 may include at least one of an application installed in the electronic device 101 and an application received from an external electronic device *(e.g.,* a sleep monitoring server 106 or the electronic device 104).

The input/output interface 140 relays a command or data input by the user through an input device (e.g., a sensor, a keyboard or a touch screen) to the processor 120, the memory 130, the communication interface 160, or the sensor module 170 through the bus 110, for example. For example, the input/output interface 140 may relay data from the user's touch input on a touch screen to the processor 120. In addition, the input/output interface 140 may output, for example, a command or data received from the processor 120, the memory 130, the communication interface 160, or the sensor module 170 through the bus 110 to an input/output device (*e.g*., a speaker or display). For example, the input/output interface 140 may output sound data from the processor 120 to the user through the speaker.

The display 150 displays various information items *(e.g.,* multimedia data or text data) to the user.

The communication interface 160 connects the electronic device 101 with external devices. For example, the communication interface 160 may be connected to the network 62 through a wireless communication or a wired communication in order for electronic device 100 to communicate with the electronic device 104 or the sleep monitoring server 106. The communication interface 160 may provide wireless communication via at least one of WiFi (IEEE 802.11), BT (Bluetooth), NFC (Near Field Communication), GPS (Global Positioning System), and cellular communication, *e.g*., LTE (Long Term Evolution), LTE-A (LTE Advanced), CDMA (Code Division Multiple Access), WCDMA (Wideband CDMA), UMTS (Universal Mobile Telecommunication System), WiBro (Wireless Broadband), or GSM (Global System for Mobile Communication). The communication interface 160 may provide wired communication via at least one of, for example, USB (Universal Serial Bus), HDMI (High Definition Multimedia Interface), RS-232 (Recommended Standard-232), and POTS (Plain Old Telephone Service).

According to one embodiment, network 162 is a telecommunication network. Network 162 may include at least one of a computer network, the Internet, or a telephone network. According to one embodiment, protocols for communication between the electronic device 101 and external devices (such as, e.g., transport layer protocol, data link layer protocol, or physical layer protocol) are supported by at least one of the applications 133, the middleware 132, the kernel 131, and the communication interface 160.

According to one embodiment, the sleep monitoring server 106 may perform and/or support at least one of the operations (or functions) implemented in the electronic device 101 when it is performing sleep monitoring.

The sensor module 170 may process at least some of information items obtained from the other components (*e.g*., the processor 120, the memory 130, the input/output interface 140, or the communication interface 160), and provide the processed information items to the user through various methods. For example, the sensor module 170 controls at least some functions of the electronic device 101 such that the electronic device 101 interworks with another electronic device (e.g., the electronic device 104 or the server 106 using the processor 120 or independently from the processor 120.

FIG 3 is a block diagram of a sensor module according to an embodiment of the present invention. Referring to FIG. 3, the sensor module 170 includes a motion sensor 210 and a biometric sensor 220.

The motion sensor 210 outputs a data value according to the motion of the electronic device 101. The motion sensor 210 includes an acceleration sensor 211, which may be a biaxial (X axis, Y axis) acceleration sensor or a triaxial (X axis, Y axis, Z axis) acceleration sensor.

The biometric sensor 220 measures various biometric signals of a human body to output various data values related to the human body. According to one embodiment, the signals from the biometric sensor 220 are used to determine whether the electronic device 101 is being currently worn on a human body.

The biometric sensor 220 includes at least a photo sensor 221, a GSR (Galvanic Skin Response) sensor 223, and a temperature sensor 225. In addition, the biometric sensor 220 may further include other biometric sensors, such as a heart rate sensor.

The photo sensor 221 converts light itself or information included in light into an electric signal. The photo sensor 221 includes a light emission unit to output light and a light reception unit to receive light. When the electronic device 101 is worn on the human body, the photo sensor 221 is positioned to be adjacent to or in contact with the human body. When the photo sensor 221 is adjacent to or in contact with a part of the human body, the light output through the light emission unit illuminates that part of the human body, and the light reflected by the illuminated part of the human body is received by the light reception unit. The photo sensor 221 measures the amount of light received through the light reception unit, and the measured amount of light may be used to determine whether the electronic device 101 is being currently worn on the human body.

The GSR sensor 223 senses a skin response to an applied electric current. The GSR sensor 223 may be an EDR (ElectroDermal Response) sensor, a PGR (Psycho Galvanic reflex) sensor, or a SCR(Skin Conductance Response) sensor. The GSR sensor 223 includes an ohmmeter to measure electrical conductivity between two points on a skin. When the electronic device 101 is worn on the human body, the GSR sensor 223 is positioned to be adjacent to or in contact with a part of the human body. When adjacent to or in contact with a part of the human body, the GSR sensor 223 can apply a predetermined small amount of current to the skin of the human body, measure an electrical conductivity between two points on the skin, and output a skin resistance value. The measured electrical conductivity may also be used for determining whether electronic device 101 is currently being worn on the human body.

The temperature sensor 225 is a sensor that measures the temperature of the human body. In one embodiment, it measures the temperature using an amount of change in internal resistance, voltage, or current. When the electronic device 101 is worn on the human body, the temperature sensor 225 is positioned to be adjacent to or in contact with a part of the human body. When adjacent to or in contact with a part of the human body, the temperature sensor 225 outputs a temperature value according to an amount of change in internal resistance, voltage or current by the human body's heat. The measured value of change in internal resistance, voltage, or current may be used for determining whether the electronic device 101 is worn on the human body.

According to various embodiments of the present invention, the biometric sensor 220 may include other sensors in addition to the photo sensor 221, the GSR sensor 223, and the temperature sensor 225. For example, the biometric sensor 220 may include a Heart Rate Variability (HRV) sensor that measures a pulse wave signal. At least one sensor included in the biometric sensor 220 should be capable of measuring a biometric signal which can be used for determining whether the electronic device 101 is currently being worn on the human body,

According to one embodiment, the electronic device calculates a motion intensity of the electronic device by comparing an output value of the motion sensor and a pre-set value for each pre-set section and, when a change in motion intensity between two or more sections corresponds to a pre-set pattern, activates the biometric sensor to determine whether the electronic device is currently being worn. If it is determined that the electronic device is currently being worn, the electronic device performs sleep monitoring.

According to various embodiments, the electronic device 101 may be any one of various devices which are wearable on a portion of the user's body.

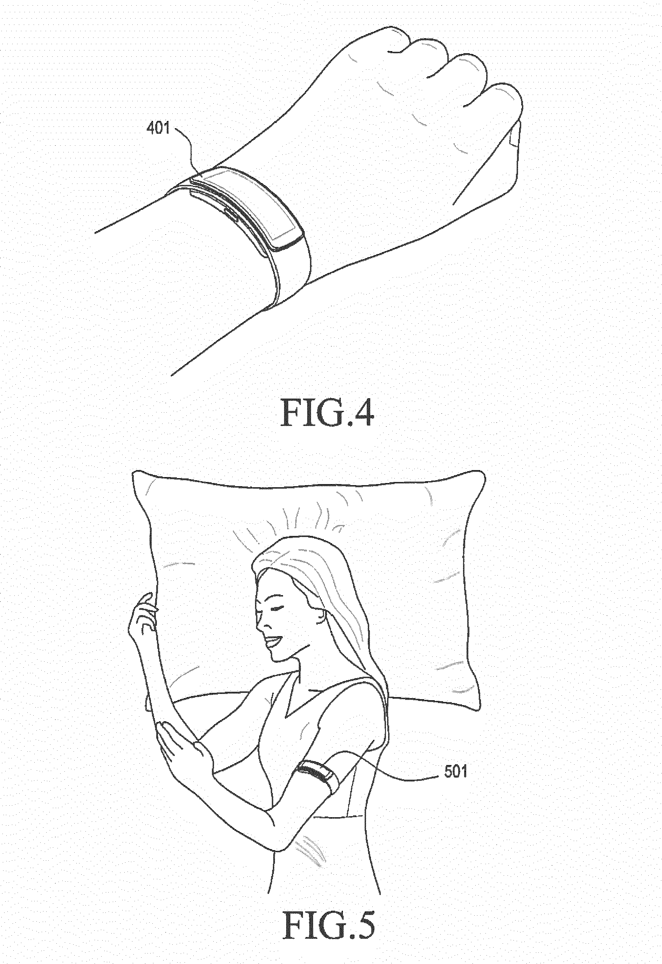

FIGS. 4 to 7 are views illustrating examples of electronic devices being worn by a user according to various embodiments of the present invention.

In FIG 4, the electronic device is a smart watch 401 which is wearable on a wrist. The smart watch 401 may calculate a motion intensity by a predetermined time unit and determine a change in motion intensity, and when the change pattern of the motion intensity corresponds to a predetermined change pattern, the smart watch 401 may determine whether the smart watch 401 is worn on the wrist. When the smart watch 401 is worn on the wrist, the smart watch 401 may execute sleep monitoring.

In FIG 5, the electronic device is an arm band 501 which is wearable on an arm. The arm band 501 may calculate a motion intensity by a predetermined time unit and determine a change in motion intensity, and when the change pattern of the motion intensity corresponds to a predetermined change pattern, the arm band 501 may determine whether the arm band 501 is worn on the arm. When the arm band 501 is worn on the arm, the arm band 501 may execute sleep monitoring.

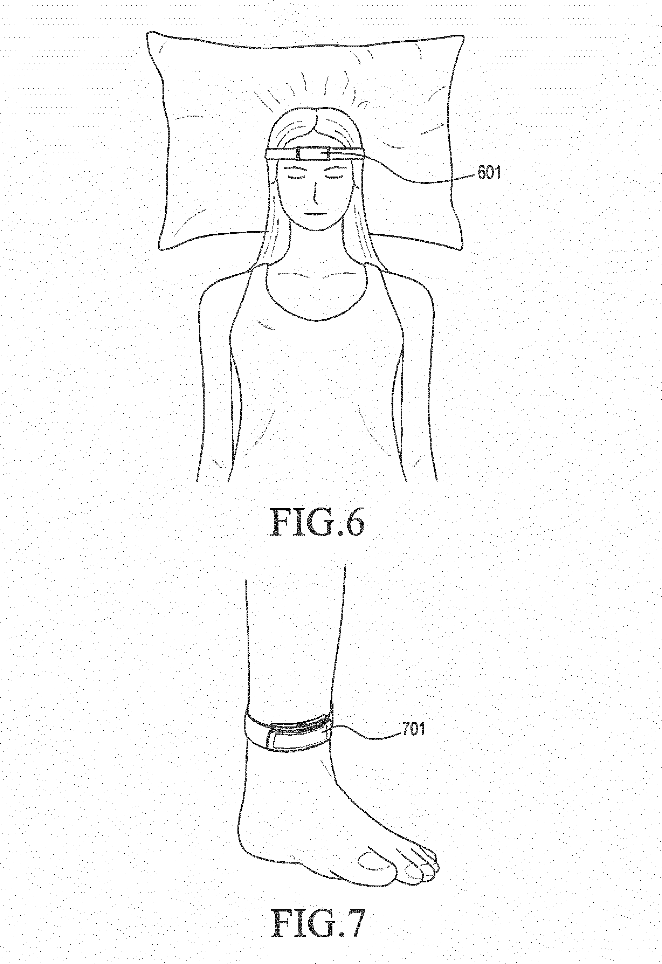

In FIG. 6, the electronic device 101 may be a hair band 601 which is wearable on head. The hair band 601 may calculate a motion intensity by a predetermined time unit and determine a change in motion intensity, and when the change pattern of the motion intensity corresponds to a predetermined change pattern, the hair band 601 may determine whether the hair band 601 is worn on a wrist. When the hair band 601 is worn on the head, the hair band 601 may execute sleep monitoring.

In FIG 7, the electronic device 101 may be an anklet 701 which is wearable on an ankle. The anklet 701 may calculate a motion intensity by a predetermined time unit and determine a change in motion intensity, and when the change pattern of the motion intensity corresponds to a predetermined change pattern, the anklet 701 may determine whether the anklet 701 is worn on the ankle. When the anklet 701 is worn on the ankle, the anklet 701 may execute sleep monitoring.

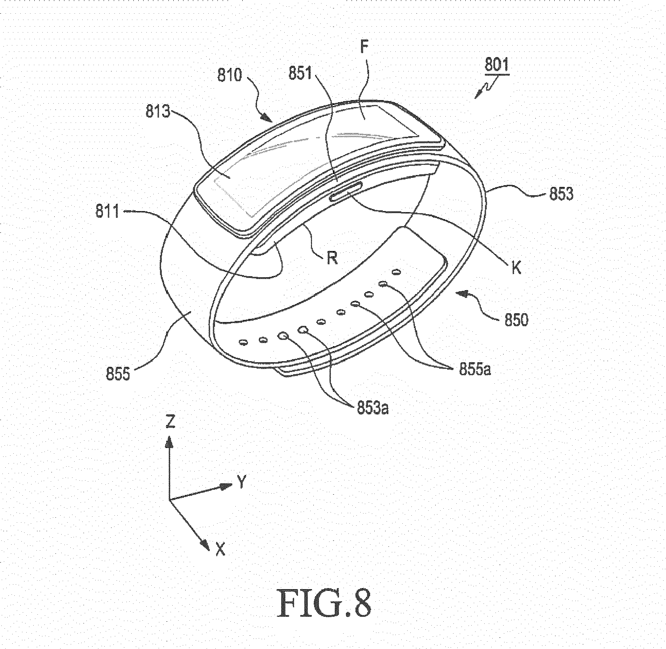

FIG. 8 is a perspective view of an electronic device according to an embodiment of the present invention. A three-dimensional X/Y/Z rectangular coordinate system is illustrated in FIG 8, in which the "Z axis" indicates the vertical direction (corresponding to the thickness of a main body 810 of the electronic device 801), the "X axis" indicates a horizontal direction (corresponding to the width of the main body 810), and the "Y" axis indicates a direction perpendicular to the other two axes (corresponding to the height of the main body 810).

Although electronic device 801, which is wearable like a watch, arm band, hair band, or anklet, is described in detail below, other embodiments of the present invention are not limited thereto, and may be implemented in the form of a bracelet, a strip, a band, an adhesion-type (plaster-type) band, a belt, an ear-wearable earphone, a headphone, a cloth, a shoe, an HMD (Head Mounted Display), a hat, a glove, a thimble (a fingertip-wearable type), a clip, a contact lens device, a digital cloth, or a remote controller.

Similarly, although the electronic device described below is applicable to a portion of the user's body having a curvature, such as, for example, a wrist, an elbow, or an ankle, embodiments of the present invention are not limited thereto, and an electronic device according to other embodiments of the present invention may be configured to be conveniently wearable on various portions of the user's body.

Electronic device 801 includes the main body 810 (functional device portion) and a wearing part (including a band or a strap) 850 including wearing members. The main body 810 may be configured to be capable of being attached to or removed from the wearing part 850. The main body 810 has a front face F, in which a display device 813 that displays various information items is disposed, a rear face R which comes into contact with the user's body when the device is worn, and a push key K on the side which is used for inputting various information items. The sensor module may be disposed on the rear face R of the main body 810.

The main body 810 has a substantially rectangular bar-type shape which at least partially has a curvature corresponding a user's body. A binding recess is formed on a side face of the main body 810, which can engage with and disengage from the wearing part 850. A plurality of binding recesses may be formed on side faces of the main body 810 or the binding recess may extend in a closed curve shape along the periphery of the main body 810.

The wearing part 850 may be made of an elastic material to allow the main body 810 to be stably worn on the user's body and/or so that the main body 810, when worn, will be in close contact with the skin of the user's body. As discussed above, the main body 810 is configured to be attached to/detached from the wearing part 850 so that the user can change wearing part 850 with other wearing parts according to the user's personality or taste. In one embodiment, a seating part 851 in the wearing part 850, by which the wearing part 850 is coupled with the main body 810, is configured to be elastically deformable, and the inner faces of first and second wearing members 853 and 855, which come into close contact with the user's body, need not be made of an elastic material. The wearing part 850 includes an opening for the main body 810 to be inserted and attached to/detached from the wearing part 850, and the seating part 851 forms the peripheral portion of the opening. When the main body 810 is coupled to the wearing part 850, at least a portion of the seating part 851 is inserted into the binding recess extending along the side faces of the main body 810.

The first and second wearing members 853 and 855 extend in opposite directions from the seating part 851 which holds the main body 810. In some embodiments, the first and second wearing members 853 and 855 may have a shape curved in consideration of the shape of the portion of the human body on which the electronic device 801 is to be worn.

Wearing part 850 includes a means for removably fastening the first and second wearing members 853 and 855 to one another. In the embodiment shown in FIG. 8, the first wearing member 853 is provided with a plurality of binding protrusions 853a and the second wearing member 855 is formed with a plurality of binding holes 855a. When the plurality of binding protrusions 853a is inserted and engaged with the plurality of binding holes 855a so as to bind the first and second wearing members 853 and 855 to one another, the wearing part 850 becomes a closed curve shape.

The plurality of binding protrusions 853a protrude inwardly, and may be formed integrally with the first wearing member 853, or manufactured as separate elements and attached to the first wearing member 853. When wearing the electronic device 801, the user selects the position of the binding holes 855a to be engaged with the first binding member 853a such that, for example, the closed curved shape of the wearing part uitably fits the size and curvature of the portion of the user's body on which the electronic device 801 is worn.

The binding structure as described above is merely one of the embodiments in accordance with the present invention, and, in other embodiments, may be replaced with other various structures (e.g., a buckle type or a hook type binding structure) according to the material and structure of the first and second wearing members 853 and 855.

The main body housing 811 of the main body 810 has a curved shape, but, since the seating part 851 is formed of a deformable elastic material, the seating part 851 may be deformed to be conformal to the shape of the main body 810 in order to be coupled to the main body 810. Because the wearing part 850 is configured to be interchangeable with other wearing parts, the wearing parts may be implemented in various designs and colors to be interchangeably used according to the user's taste. That is, the wearing part 850 may be utilized as an accessory that may show the user's own unique personality.

Since the curvatures of the individual users' body parts are different from each other, the feelings of wearing sensed by the individual users will be different when the users wear electronic devices of the same shape. For example, since a woman's wrist is usually thinner than a man's wrist, it may be difficult to provide convenient feelings of wearing to all the users when the users wear the same size of body-wearable electronic devices. However, according to embodiments of the present invention, the electronic device 801 has a flexible structure, because the main body 810 and the wearing part 850 can be attached to/detached from each other. Accordingly, the user may select a wearing part 850 suitable for the user's own body characteristics so that the electronic device can be conveniently worn.

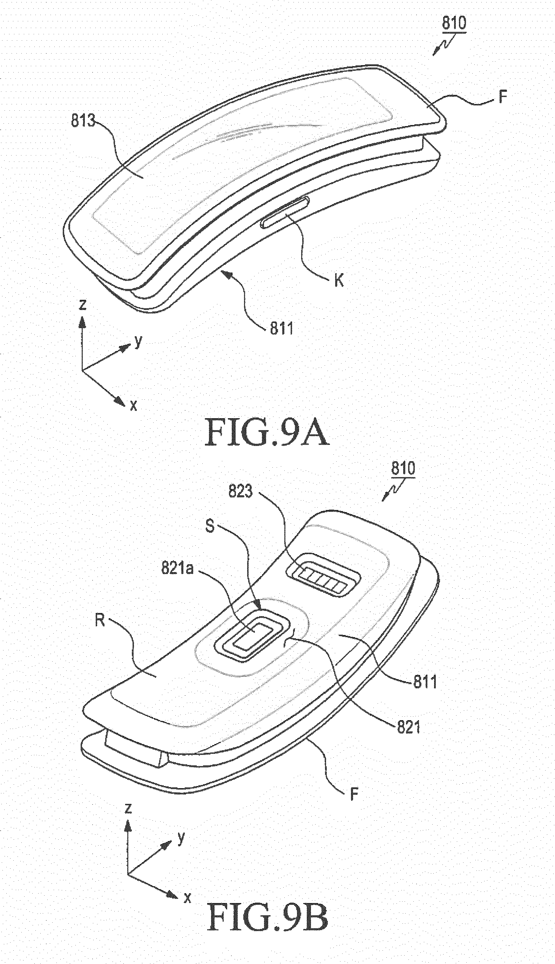

FIG. 9A is a perspective view of the front of the main body of an electronic device according to an embodiment of the present invention, and FIG. 9B is a perspective view of the rear of the main body of the electronic device.

Referring to FIGS. 9A and 9B, the main body 810 has a rectangular bar-type shape having a curvature and elongated in the height direction Y, in which the main body 810 includes a main body housing 811. The main body housing 811 includes a front face F, on which display device 813 is mounted, and a rear face R, which is in contact with the user's body when the device is worn. The main body 810 may further include pushing keys for input various information items, such as, *e.g.,* the side key K. The front face F has a first curvature and the rear face R has a second curvature, in which the first and second curvature may be determined in consideration of product design, sizes and curvatures of various users' wrists, and various users' feelings when wearing the electronic device. The embodiment shown in FIGS. 9A-9B has a configuration in which the first curvature is smaller than the second curvature.

The screen of display device 813 in the front face F of the main body housing 811 should be configured so that the display device 813 is convenient to watch, and the rear face R should provide a shape so that a sensor module S (e.g., biometric sensor) disposed therein will be in close contact and comfortable with the user's wrist.

The main body 810 is configured to be thickest at the center and then the thickness is gradually reduced from the middle portion toward the opposite ends in the height direction Y. It has been described in this embodiment that the rear face R has a second curvature, but the rear face R may be configured as a substantially flat face, or may have a partially flat face which does not have a curvature.

The sensor module S provided disposed within the main body 810 may include at least one of a photo sensor, a GSR sensor, a temperature sensor, and a heart rate sensor. In addition, the sensor module S may include other sensors that detect whether the electronic device 801 is worn. Although the display device 813 in FIG. 9A-9B is curved to reflect the curvature of where the device is worn on the user's body, the display device 813 may be constituted with a flat display, a curved display, or a flexible display, each or any of which may be a Liquid Crystal Display (LCD) or an Organic Light Emitting Diode (OLED) display.

The sensor module S includes a sensor interface unit 821a, an interface window, that is disposed on the rear face R of the main body 811. The sensor interface unit 821a is disposed on protrusion 821, so that it may come in contact with the user's body more closely in sensing a biometric signal. Charging terminals are arranged as connection members 823 on the rear face R of the main body 811. The arrangement of the connection members 823 may be positioned to be adjacent to the sensor module S.

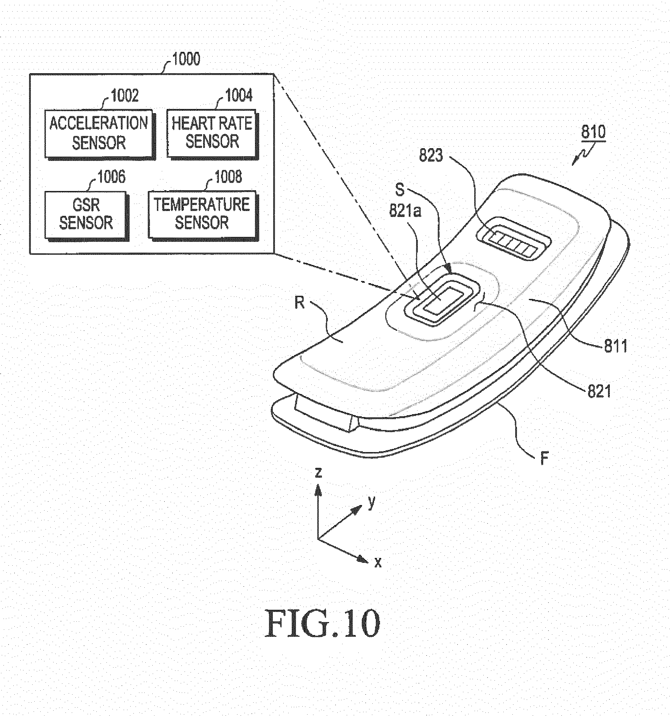

FIG 10 has an expanded view of a sensor module on the rear of the main body of the electronic device according to an embodiment of the present invention. Referring to FIG 10, the sensor module 1000 is provided on the rear face R of the main body housing 810 in a modular form that includes an acceleration sensor 1002 and biometric sensors for measuring biometric signals, in this example, a heart rate sensor 1004, a GSR sensor 1006, and a temperature sensor 1008. The acceleration sensor 1002 may be a bi-axis (X axis, Y axis) acceleration sensor or a tri-axis (X axis, Y axis, Z axis) acceleration sensor. The biometric sensors measure various biometric signals of a human body to output various biometric sensor measurements related to the human body, and at least one of the biometric sensors may be used to detect a wearing state (i.e., whether the device is being worn by a user). According to various embodiments, , the heart rate sensor 1004, the GSR sensor 1006, the temperature sensor 1008, two or more of those sensors, or another biometric sensor may be used to detect the wearing state.

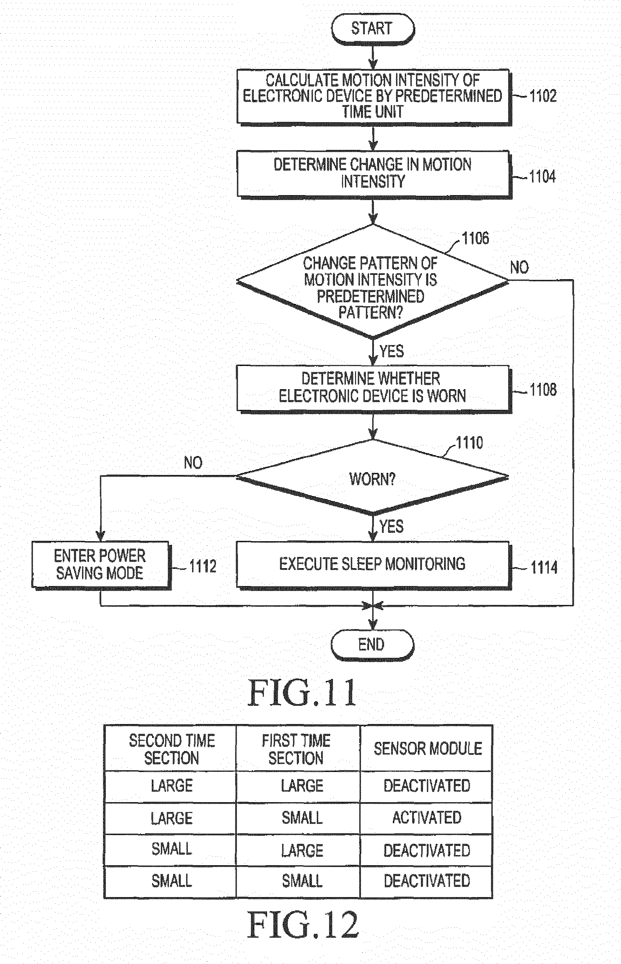

FIG. 11 is a flowchart of a method of sleep monitoring in an electronic device according to an embodiment of the present invention. Referring to FIG. 11, in step 1102, the electronic device 101 calculates the motion intensity of the electronic device 101 over a predetermined period of time. According to an embodiment, the electronic device 101 obtains an acceleration value according to the motion of the electronic device 101 using the acceleration sensor 210 and calculates the motion intensity of the electronic device 101 using the acceleration value. According to an embodiment, the predetermined period of time may be selected from 30 seconds, 1 minute, and/or 2 minutes. According to an embodiment, the electronic device 101 calculates the motion intensity 10 times per second, 20 times per second, or in a predetermined motion calculation cycle. According to various embodiments, the motion intensity calculation cycle may be variable.

In step 1104, the electronic device 101 determines whether there is a change in motion intensity. According to one embodiment, the electronic device 101 determines the change in motion intensity by comparing the motion intensity for the present time section and the motion intensity for the previous time section.

In step 1106, the electronic device 101 determines whether the pattern of motion intensity change is the same as a predetermined pattern. According to one embodiment, the predetermined pattern is a pattern in which the motion intensity is changed from a small value to a large value. When the change pattern does not fit a predetermined pattern in step 1106, the method ends.

When the motion intensity change pattern fits a predetermined pattern in step 1106 (such as, e.g., when the motion intensity changes from a small value to a large value), the electronic device 101 determines whether the electronic device 101 is currently being worn in step 1108. According to one embodiment, the electronic device 101 determines whether the electronic device 101 is currently being worn using the biometric sensor 220.

According to one embodiment, the electronic device 101 determines whether the electronic device 101 is worn using the measured amount of light from the photo sensor 221. According to one embodiment, the electronic device 101 determines whether the electronic device 101 is worn using electrical conductivity measured by the GSR sensor 223. According to one embodiment, the electronic device 101 determines whether the electronic device 101 is worn using the temperature measured by the temperature sensor 225.

If it is determined that the electronic device 101 is not currently being worn in step 1110, the electronic device 101enters a power saving mode in step 1112. If it is determined that the electronic device 101 is currently being worn in step 1110, the electronic device 101 performs sleep monitoring in step 1114.

According to various embodiments of the present invention, the electronic device 101 performs sleep monitoring after determining if there has been a change in motion intensity between a current first time section and a previous second time section, which is a time section prior to the current first time section.

FIG. 12 is a table of change patterns in motion intensity between a first time, which is the current time, and a second time, which is the time prior to the current time, according to a first embodiment.

Referring to FIG. 12, the change in motion intensity between the first time section and the second time section may be any one of four patterns. The first pattern corresponds to a case in which both the motion intensities in the first time section and the second time section are a large value (Large), the second pattern corresponds to a case in which the motion intensity in the first time section is a large value (Large) and the motion intensity in the second time section is a small value (Small). The third pattern corresponds to a case in which the motion intensity in the first time section is a small value (Small), and the motion intensity in the second time section is a large value (Large). The fourth pattern corresponds to a case in which both the motion intensities in the first time section and the second time section are a small value (Small).

According to one embodiment, the determination as to whether the motion intensity is a large or small value may be made based on a predetermined threshold value. According to this embodiment, when the motion intensity is a value equal to or larger than the predetermined threshold value, the motion intensity is the large value, and when the motion intensity is a value smaller than the predetermined threshold value, the motion intensity is the small value.

As shown in FIG. 12, when the motion intensities in both the first and second time sections are the large value (Large), or when the motion intensity in the first time section is the small value (Small) and the motion intensity of the second time section is the large value (Large), or when the motion intensities in both the first and second time sections are the small value (Small), the sensor module is deactivated, and when the motion intensity in the first time section is the small value (Small) and the motion intensity in the second time section is the large value (Large), the sensor module is activated. In other words, the Small - Large pattern is the predetermined pattern that causes the electronic device to next determined whether it is being worn or not.

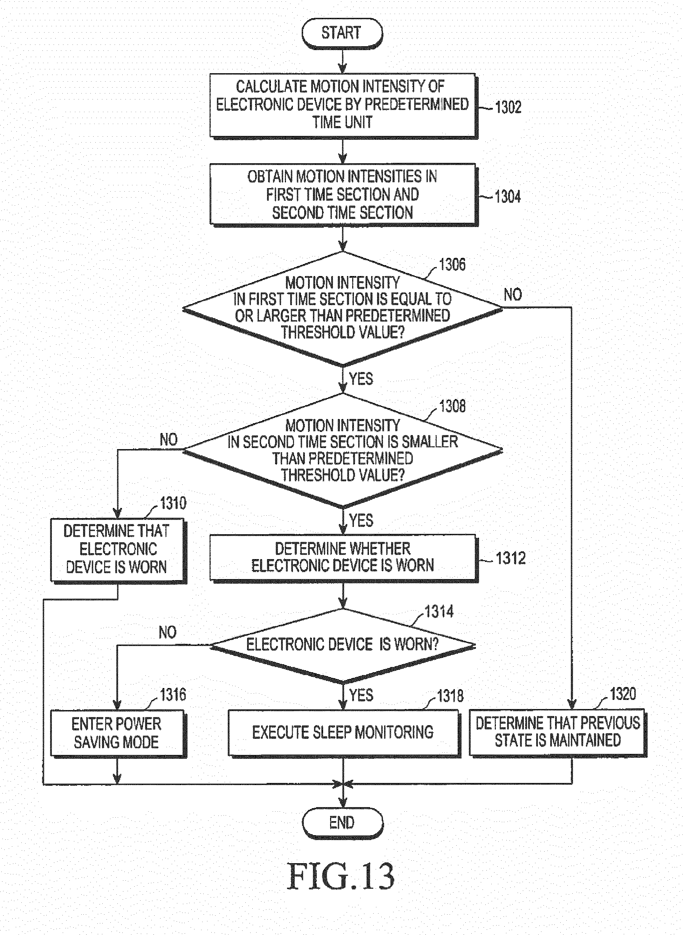

FIG. 13 is a flowchart of a method of sleep monitoring according to the first embodiment.

Referring to FIG. 13, in step 1302, the electronic device 101 calculates the motion intensity of the electronic device 101 by a predetermined time unit. According to one embodiment, the electronic device 101 obtains an acceleration value using the acceleration sensor 210 and calculates the motion intensity using the acceleration value by the predetermined time unit. The predetermined time unit may be selected from various periods such as 30 seconds, 1 minute, and 2 minutes. The electronic device 101 may calculate the motion intensity ten times per second, twenty times per second, or in a predetermined motion calculation cycle. According to various embodiments, the motion intensity calculation cycle may be variable.

In step 1304, the electronic device 101 obtains the motion intensity in the first time section and the motion intensity in the second time section. According to an embodiment in which the predetermined time unit is 1 minute, the electronic device 101 obtains the motion intensity for the most recent 1 minute and the motion intensity for the previous minute, i.e., between 1 minute ago and 2 minutes ago.

In step 1306, the electronic device 101 determines whether the motion intensity in the first time section is a value equal to or larger than the predetermined threshold value. When the motion intensity in the first time section is a value equal to or larger than the predetermined threshold value, the motion intensity is the large value.

When the motion intensity in the first time section is equal to or larger than the predetermined threshold value in step 1306, the electronic device 101 determines whether the motion intensity in the second time section is a value smaller than the predetermined threshold value in step 1308. When the motion intensity in the first or second time section is a value smaller than the predetermined threshold value, the motion intensity is the small value, and when the motion intensity in the first or second time section is equal to or larger than the predetermined threshold value, the motion intensity is the large value.

When the motion intensity in the first time section is the large value in step 1306 and the motion intensity in the second time section is the large value in step 1308, the electronic device 101 determines in step 1310 that the electronic device 101 is currently being worn.

When the motion intensity in the first time section is the large value in step 1306 and the motion intensity in the second time section is the small value in step 1308, the electronic device 101 determines in step 1312 whether electronic device 101 is currently being worn or not. The electronic device 101 determines whether the electronic device 101 is currently being worn using a biometric sensor, such as, e.g., photo sensor 221, GSR sensor 223, and/or temperature sensor 225.

If it is determined in step 1314 that the electronic device 101 is not currently being worn, the electronic device 101 enters a power saving mode in step 1316. If it is determined in step 1314 that the electronic device 101 is currenly being worn, the electronic device 101 performs sleep monitoring in step 1318.
In step 1306, if the motion intensity in the first time section is neither equal to nor larger than the predetermined threshold value, the electronic device 101 determines that the motion intensity in the first time section is the small value, and the electronic device 101 determines in step 1320 that the previous state of electronic device 101 is maintained According to this embodiment, when the motion intensity in the first time section is the small value, the electronic device 101 determines that the previous state is maintained regardless of the value of the motion intensity in the second time section. The previous state may be a state where the electronic device is being worn, a state in which the electronic device is in the power saving mode without being worn, or a state in which the electronic device is being worn and sleep monitoring is performed.

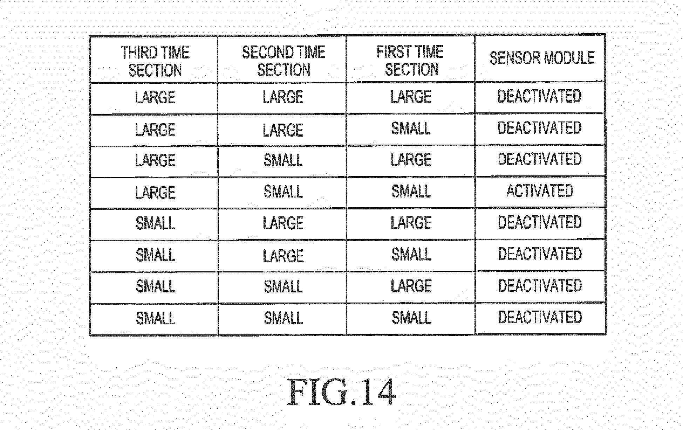

FIG 14 is a table of change patterns in motion intensity over three consecutive time periods: a first time section, which is a current time section, a second time section, which is a time section prior to the current time section, and a third time section, which is a time section prior to the second time section, according to the second embodiment.

Referring to FIG 14, the change in motion intensity over the three time sections may be any one of eight patterns. According to this embodiment, the first pattern corresponds to when the motion intensities of all three time sections are the large value (Large), and the second pattern corresponds to when the motion intensities of the first and second time sections are the large value (Large) and the motion intensity of the third time section is the small value (Small). The third pattern corresponds to when the motion intensity in the first time section is the large value (Large), the motion intensity in the second time section is the small value (Small), and the motion intensity in the third time section is the large value (Large). The fourth pattern corresponds to when the motion intensity in the first time section is the large value (Large) and the motion intensities in both the second and third time sections are the same value (Small). The fifth pattern corresponds to when the motion intensity in the first time section is the small value (Small) and the motion intensity in both the second and third time sections are the large value (Large). The sixth pattern corresponds to when the motion intensity in the first time section is the small value (Small), the motion intensity in the second time section is the large value (Large), and the motion intensity in the third time section is the small value (Small). The seventh pattern corresponds to when the motion intensities in both the first and the second time sections are the small value (Small) and the motion intensity of the third time section is the large value (Large). The eighth pattern corresponds to when the motion intensity of all three time sections is the small value (Small).

According to this embodiment, when the motion intensity in the first time section is the large value (Large) and the motion intensity in both the second and third time sections are the small value (Small), the sensor module that detects whether the electronic device is worn or not is activated, and in the other cases, the sensor module that detects whether the electronic device is worn or not is deactivated.

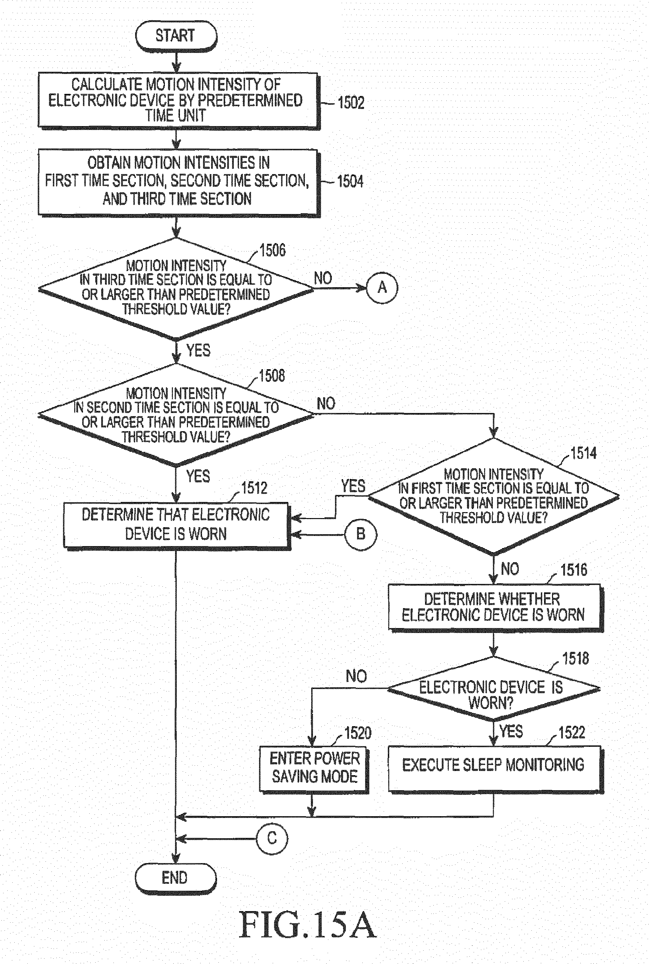

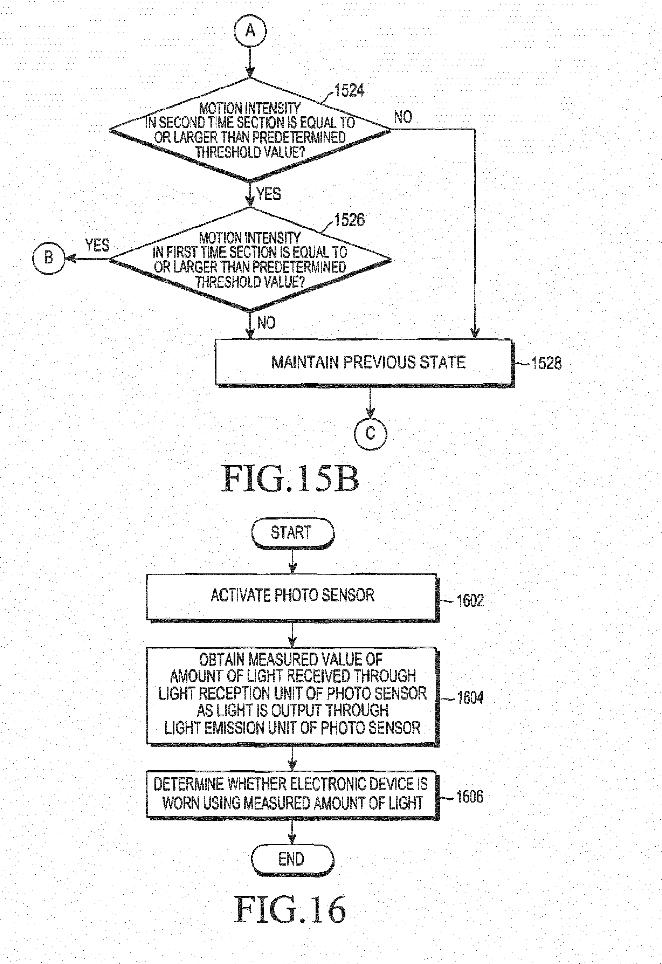

FIGS. 15A and 15B comprise a flowchart of a method of sleep monitoring according to pattern changes in motion intensity over three time periods, according to the second embodiment.

Referring to FIG 15A, the electronic device 101 calculates the motion intensity of the electronic device 101 by predetermined time units in step 1502. According to one embodiment, the electronic device 101 calculates the motion intensity of the electronic device 101 using the acceleration value from acceleration sensor 210 each predetermined time unit. According to various embodiments, the predetermined time unit may be 30 seconds, 1 minute, or 2 minutes, and the electronic device 101 calculates the motion intensity 10 times per second, 20 times per second, or in a predetermined or variable motion intensity calculation cycle.

In step 1504, the electronic device 101 obtains the motion intensities over three consecutive time units: the first time section, which is the current time section, the second time section, which is the time section prior to the current time section, and the third time section, which is the time section prior to the second time section. According to an embodiment where the predetermined time unit is 1 minute, the three consecutive time sections are the most recent 1 minute, between one minute ago and two minutes ago, and between two minutes ago and three minutes ago.

In step 1506, the electronic device 101 determines whether the motion intensity in the third time section is equal to or larger than the predetermined threshold value. When the motion intensity in the third time section is equal to or larger than the predetermined threshold value, the electronic device 101 determines that the motion intensity in the third time section is the large value.

When the motion intensity in the third time section is the large value, i.e., equal to or larger than the predetermined threshold value, the electronic device 101 determines in step 1508 whether the motion intensity in the second time section is equal to or larger than the predetermined threshold value. When the motion intensity in the second time section is equal to or larger than predetermined threshold value, the electronic device 101 determines that the motion intensity in the second time section is the large value.

When the motion intensity in the third time section is equal to or larger than the predetermined threshold value in step 1506 and the motion intensity in the second time section is equal to or larger than the predetermined threshold value in step 1508" the electronic device 101 determines whether the motion intensity in the first time section is equal to or larger than the predetermined threshold value in step 1510. When the motion intensity in the first time section is equal to or larger than predetermined threshold value, the electronic device 101 determines that the motion intensity of the first time section is the large value.

When the motion intensity in the third time section is the large value in step 1506, the motion intensity in the second time section is the large value in step 1508, and the motion intensity in the first time section is the large value in step 1510, or when the motion intensity in the third time section is the large value in step 1506, the motion intensity in the second time section is the large value in step 1508, and the motion intensity in the first time section is the small value in step 1510, the electronic device 101 determines in step 1512 that the electronic device 101 is currently being worn.

When the motion intensity in the third time section is the large value, i.e., equal to or larger than the predetermined threshold value in step 1506 and the motion intensity in the second time section is the small value, i.e., neither equal to nor larger than the predetermined threshold value in step 1508, the electronic device 101 determines in step 1514 whether the motion intensity in the first time section is equal to or larger than the predetermined threshold value.

When the motion intensity in the third time section is the large value, i.e., equal to or larger than the predetermined threshold value in step 1506, the motion intensity in the second time section is the small value, i.e., neither equal to nor larger than the predetermined threshold value in step 1508, and the motion intensity in the first time section is the large value, i.e., equal to or larger than the predetermined threshold value, the electronic device 101 determines that the electronic device 101 is currently being worn in step 1512.

When the motion intensity in the third time section is the large value, i.e., equal to or larger than the predetermined threshold value in step 1506, the motion intensity in the second time section is the small value, i.e., neither equal to nor larger than the predetermined threshold value, in step 1508, and when the motion intensity in the first time section is the small value, i.e., neither equal to nor larger than the predetermined threshold value, in step 1514, the electronic device 101 determines whether or not the electronic device is currently being worn in step 1516.

As discussed above, the electronic device 101 may determine whether the electronic device 101 is currently being worn using, e.g., the photo sensor 221, the GSR sensor 223, and/or the temperature sensor 225. If, in step 1518, the electronic device 101 is not currently being worn, the electronic device 101 enters a power saving mode in step 1520. If, in step 1518, the electronic device 101 is currently being worn, the electronic device 101 performs sleep monitoring in step 1522.

If, in step 1506 of FIG 15A, the motion intensity in the third time section is neither equal to nor larger than the predetermined threshold value, the electronic device 101 in step 1524 of FIG. 15B determines whether the motion intensity in the second time section is equal to or larger than the predetermined threshold value. If the motion intensity in the second time section is equal to or larger than the predetermined threshold value, the motion intensity is the large value, and if the motion intensity in the second time section is neither equal to nor larger than the predetermined threshold value, the motion intensity is the small value.

When the motion intensity in the third time section is the small value, i.e., neither equal to nor larger than the predetermined threshold value, in step 1506, and the motion intensity in the second time section is the large value, i.e., equal to or larger than predetermined threshold value, in step 1524, the electronic device 101 determines in step 1526 whether the motion intensity in the first time section is equal to or larger than the predetermined threshold value. When the motion intensity in the first time section is equal to or larger than the predetermined threshold value, the motion intensity is the large value, and when the motion intensity in the first time section is neither equal to nor larger than the predetermined threshold value, the motion intensity is the small value.

When the motion intensity in the third time section is the small value, i.e., neither equal to nor larger than the predetermined threshold value, in step 1506, the motion intensity in the second time section is the large value, i.e., equal to or larger than the predetermined threshold value, in step 1524, and the motion intensity in the first time section is the large value, i.e., equal to or larger than the predetermined threshold value, in step 1526 of FIG. 15B, the electronic device 101 determines that the electronic device 101 is currently being worn in step 1512 of FIG. 15A.

When the motion intensity in the third time section is the small value, i.e., neither equal to nor larger than the predetermined threshold value, in step 1506, the motion intensity in the second time section is the large value, i.e., equal to or larger than the predetermined threshold value, in step 1524, and the motion intensity in the first time section is the small value, i.e., neither equal to nor larger than the predetermined threshold value, in step 1526, the electronic device 101 maintains the previous state in step 1528.

When the motion intensity in the third time section is the small value, i.e., neither equal to nor larger than the predetermined threshold value, in step 1506, and the motion intensity in the second time section is the small value, i.e., neither equal to nor larger than the predetermined threshold value, in step 1526, the electronic device 101 determines that the previous state is maintained in step 1528. According to this embodiment, when the motion intensity in both the third and second time sections is the value, i.e., neither equal to nor larger than the predetermined threshold value, the electronic device 101 determines that the previous state is maintained regardless of the value of the motion intensity in the first time section. The previous state may be when the electronic device is being worn, when the electronic device is in the power saving mode without being worn, or when sleep monitoring is performed while the electronic device is being worn.

FIG. 16 is a flowchart of a method of determining whether the electronic device is worn using the photo sensor according to an embodiment of the present invention. Referring to FIG 16, the electronic device 101 activates the photo sensor in step 1602 so as to determine whether the electronic device is worn or not.

In step 1604, the electronic device 101 outputs light through the light emission unit of the photo sensor 221 and obtains the value of the measured amount of light received through the light reception unit of the photo sensor 221.

Using the measured amount of light, in step 1606, the electronic device 101 determines whether the electronic device 101 is currently being worn. The electronic device 101 determines whether the photo sensor 221 is positioned adjacent to or in contact with a part of the human body according to the measured amount of light, and using the information as to whether the photo sensor 221 is positioned adjacent to or in contact with a part of the human body, the electronic device 101 determines whether the electronic device 101 is currently being worn on the human body.

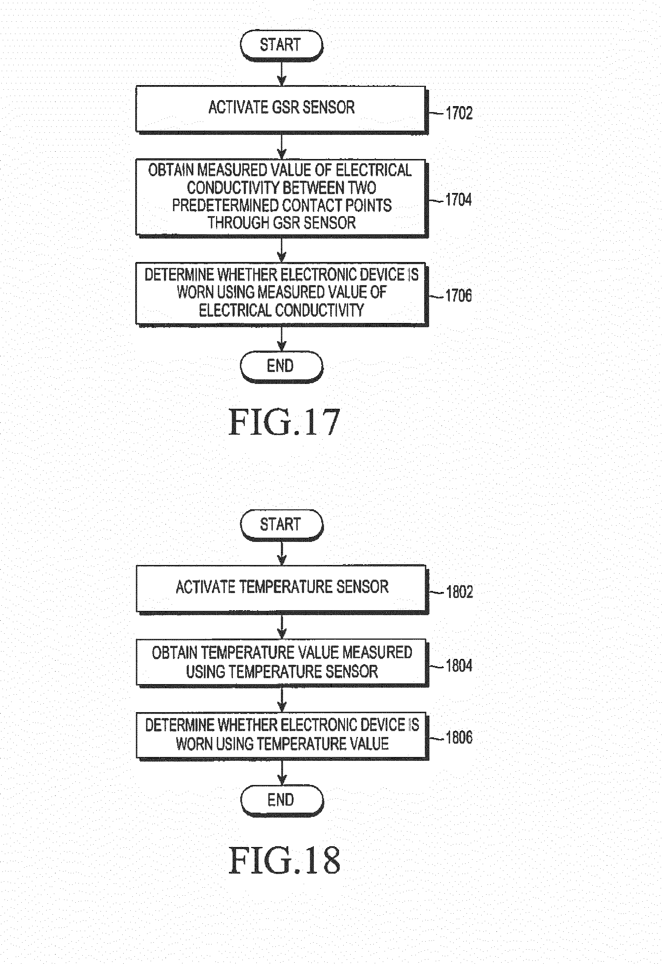

FIG. 17 is a flowchart of a method of determining whether the electronic device is currently being worn using the GSR sensor according to an embodiment of the present invention. Referring to FIG. 17, the electronic device 101 activates the GSR sensor 223 in step 1702 so as to determine whether the electronic device 101 is currently being worn.

In step 1704, the electronic device 101 obtains measured value of electrical conductivity between two predetermined contacts using the GSR sensor 223.

In step 1706, the electronic device 101 obtains a skin resistance using the measured value of the electrical conductivity, and determines whether the electronic device 101 is worn according to the skin resistance. The electronic device 101 determines whether the GSR sensor 223 is positioned adjacent to or in contact with a part of the human body according to the measured value of the electrical conductivity, and using the information as to whether the GSR sensor 223 is positioned adjacent to or in contact with a part of the human body, the electronic device 101 determines whether the electronic device 101 is currently being worn on the human body.

FIG. 18 is a flowchart of a method of determining whether the electronic device is currently being worn using the temperature sensor according to an embodiment of the present invention. Referring to FIG. 18, the electronic device 101 activates the temperature sensor 225 in step 1802 so as to determine whether the electronic device 101 is currently being worn.

In step 1804, the electronic device 101 obtains a temperature value measured through the temperature sensor 225.

In step 1806, the electronic device 101 determines whether the electronic device 101 is currently being worn using the measured temperature value. The electronic device 101 determines whether the temperature sensor 225 is positioned adjacent to or in contact with a part of the human body according to the measured temperature value, and using the information as to whether the temperature sensor 225 is positioned adjacent or in contact with a part of the human body, the electronic device 101 determines whether the electronic device 101 is worn on the human body.

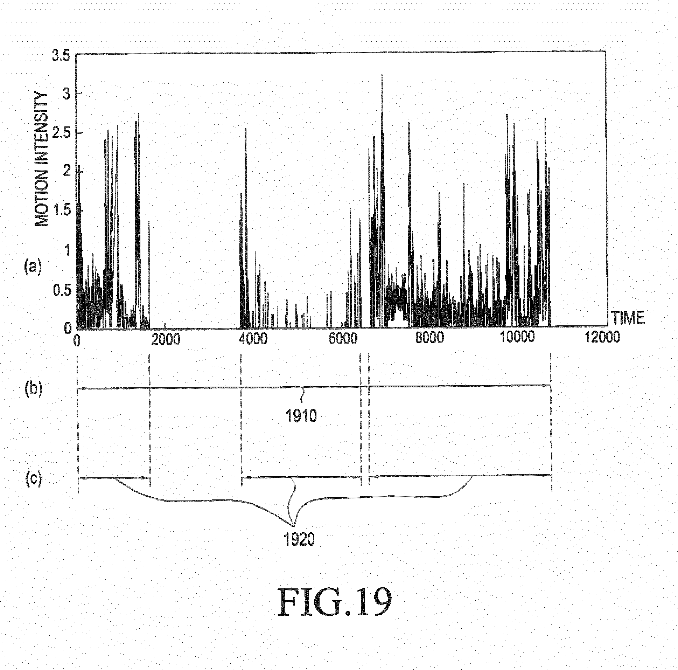

FIG. 19 is a graph of motion intensity over time according to an embodiment of the present invention. Referring to FIG. 19, the horizontal (X) axis is the time axis, and the vertical (Y) axis is the motion intensity axis. (a) in FIG. 19 is the graph of motion intensity over time, (b) is a section in which the sensor module would be activated according to a predetermined time period in a conventional sleep monitoring method, and (c) is a section showing when the sensor module is activated based on the pattern of motion intensity change fits a predetermined change pattern according to an embodiment of the present embodiment. Upon comparing (b) and (c), it can be seen that when the sensor module is activated when the pattern of motion intensity change is the predetermined change pattern (as shown by 1920) rather than being activated according to the predetermined time period (as shown by 1910), battery consumption is reduced since the sensor module is activated only when it is necessary.

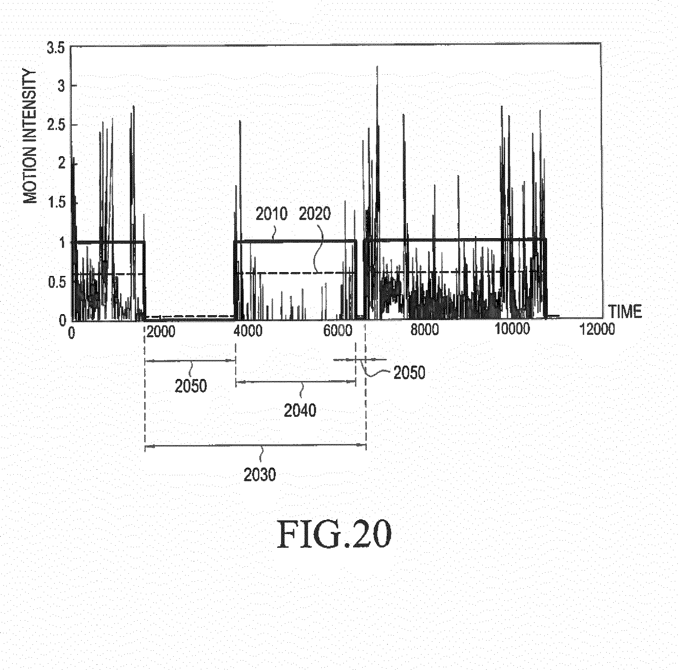

FIG. 20 is a graph showing the result of determining whether the electronic device is worn according to an embodiment of the present invention. Referring to FIG. 20, the horizontal (X) axis is a time axis, and the vertical (Y) axis is a motion intensity axis. Reference numeral 2010 represents a result of determining whether the electronic device is worn when the sensor module is activated according to a predetermined time period, and reference numeral 2020 represents a result of determining whether the electronic device is worn when the sensor module is activated according to whether a change pattern of motion intensity is a predetermined change pattern. Upon comparing 2010 and 2020, it can be confirmed that the results of determining whether the electronic device is worn are the same in the case where the sensor module is activated according to the predetermined time period and in the case where the sensor module is activated when the change pattern of motion intensity is the predetermined change pattern. Meanwhile, in the prior art, since the sleep monitoring is not performed according to the result of determining whether the electronic device is worn, a section (2050) in which the electronic device is not worn included in the section indicated by reference 2030 may be determined as a sleep section. However, in the present embodiment, since the sleep section is determined by performing the sleep monitoring only when the electronic device is worn as in the section indicated by reference numeral 2040 so as to determine the sleep section, the sleep section may be more accurately determined compared to the prior art.

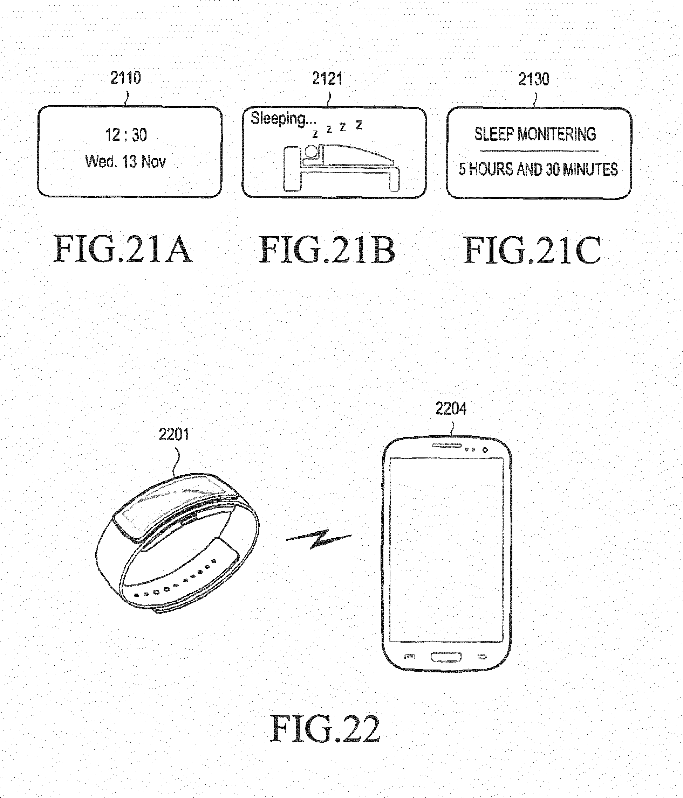

FIGS. 21A, 21B, and 21C illustrate examples of sleep monitoring screens of the electronic device according to an embodiment of the present invention. In FIG 21A, the electronic device 101 displays a basic screen 2110 including current time, date, day of the week. FIG 21B shows when execution of the sleep monitoring is started, and a sleep monitoring screen 2121 is displayed indicating that the sleep monitoring is being executed. FIG 21C shows when the execution of the sleep monitoring is terminated, and the electronic device 101 displays a sleep monitoring result screen 2130 indicating a result of sleep monitoring. On the sleep monitoring result screen 2130, the total sleep time is displayed. According to one embodiment, the sleep start time and the sleep termination time may also be displayed on the sleep monitoring result screen 2130.

According to various embodiments of the present invention, the electronic device 101 may be connected with an external electronic device 104 through a communication means, and may be operated by a control command of the external electronic device 104. According to one embodiment, the electronic device 101 may be the external electronic device on the position of the electronic device 104.

FIG. 22 is a view illustrating an electronic device 2201 and an external electronic device 2204 according to an embodiment of the present invention. Referring to FIG 22, the electronic device 2201 may be a device wearable on a part of a body, such as a wrist, an arm, a head, or an ankle, and the external electronic device 2204 may be a portable terminal capable of communicating with the electronic device 2201.

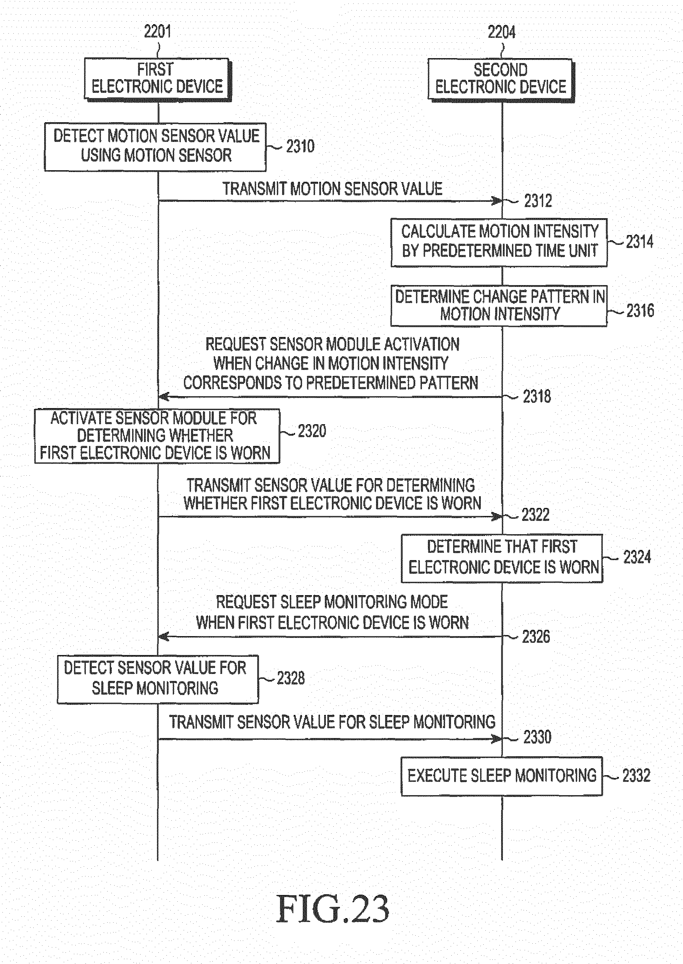

FIG. 23 is a flowchart of sleep monitoring operations using the electronic device 2201 and the external electronic device 2204 according to an embodiment of the present invention. Referring to FIG 23, in step 2310, the electronic device 2201 (hereinafter, referred to as a "first electronic device") detecta a motion of the electronic device 2201 using a motion sensor. According to one embodiment, the motion sensor may be an acceleration sensor.

In step 2312, the first electronic device 2201 transmits the sensor value detected by the motion sensor to the external electronic device 2204 (hereinafter, referred to as a "second electronic device").

In step 2314, the second electronic device 2204 calculates the motion intensity of the first electronic device 2201 over a predetermined time period using the sensor value received from the first electronic device 2201. According to one embodiment, the predetermined time period may be, for example, 30 seconds, 1 minute, and/or two minutes. According to one embodiment, the sensor value may be an acceleration sensor value, and the motion intensity may be calculated 10 times per second, 20 times per second, or in a predetermined and/or variable motion calculation cycle.

In step 2316, the second electronic device 2204 determines a change pattern of motion intensity. According to one embodiment, the second electronic device 2204 determines the change pattern of motion intensity by comparing the motion intensity for the current time section and the motion intensity for the previous time section, and then determines whether the change pattern of motion intensity is a predetermined pattern. According to one embodiment, the predetermined pattern is a pattern in which the motion intensity is changed from a small value to a large value.

When the change pattern of motion intensity is the predetermined pattern in step 2316, the second electronic device 2204 requests activation of the sensor module of the first electronic device 2201 in step 2318. In step 2320, the first electronic device 2201 activates the sensor module for determining whether the first electronic device 2201 is worn. According to various embodiments, the sensor capable of determining whether the first electronic device 2201 is worn may include at least one of a photo sensor, a GSR sensor, and a temperature sensor.

The first electronic device 2201 detects the sensor value using one or more sensors capable of determining whether the first electronic device 2201 is worn, and in step 2322, the first electronic device 2201 transmits the sensor value for determining whether the first electronic device is worn to the second electronic device 2204. According to one embodiment, the first electronic device 2201 measures an amount of light which is output through the light emission unit of the photo sensor and then received through the light reception unit of the photo sensor, and transmits the measured amount of light to the second electronic device 2204. According to one embodiment, the electronic device 2201 measures an electrical conductivity through a GSR sensor and transmits the measured electrical conductivity to the second electronic device 2204. According to one embodiment, the electronic device 2201 measures a temperature through the temperature sensor and transmit the measured temperature value to the second electronic device 2204.

In step 2324, the second electronic device 2204 determines whether the first electronic device 2201 is currently being worn, based on the received sensor value from the first electronic device 2201, which may be, for example, a measured amount of light, a measured electrical conductivity, and/or a measured temperature.

If it is determined that the first electronic device 2201 is currently being worn in step 2324 (which, in FIG. 23, it is), the second electronic device 2204 requests that the first electronic device 2201 operate in sleep monitoring mode in step 2326.

According to the received request from the second electronic device 2204, the first electronic device 2201 enters the sleep monitoring mode and in step 2328 detects a sensor value for sleep monitoring. According to one embodiment, the sensor value for sleep monitoring may be a motion sensor value, for example, an acceleration sensor value. In step 2330, the first electronic device 2201 transmits the detected sensor value for sleep monitoring to the second electronic device 2204.

In step 2332, the second electronic device 2204 performs sleep monitoring usingsensor values for sleep monitoring received from the first electronic device 2201.

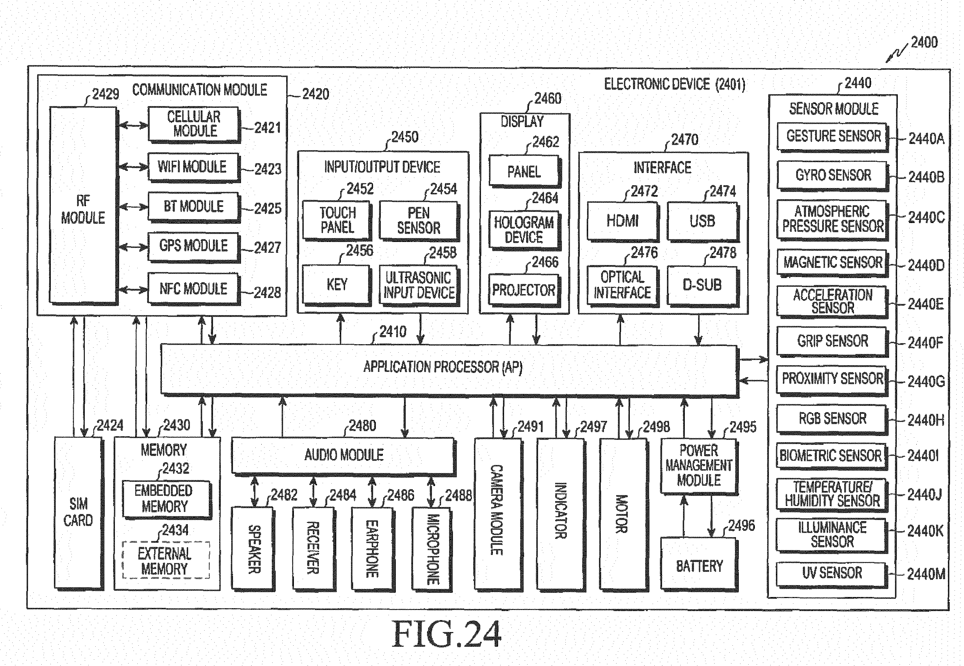

FIG. 24 is a block diagram of an electronic device according to an embodiment of the present invention. Referring to FIG. 24, electronic device 2401 may comprise the entirety or a part of the electronic device 101 or the electronic device 104 illustrated in FIG. 2. The electronic device 2401 includes an Application Processor (AP) 2410, a communication module 2420, a Subscriber Identification Module (SIM) card 2424, a memory 2430, a sensor module 2440, an input/output device 2450, a display 2460, an interface 2470, an audio module 2480, a camera module 2491, a power management module 2495, a battery 2496, an indicator 2497, and a motor 2498.

The AP 2410 may drive an operating system or an application program to control a plurality of hardware or software components connected to the AP 2410, and to perform processing and arithmetic operation of various data including multimedia data. The AP 2410 may be implemented by, for example, a System on Chip (SoC). According to one embodiment, the AP 2410 may further include a Graphic Processing Unit (GPU) (not illustrated).

The communication module 2420 performs data transmission/reception communication with other electronic devices (*e.g.*, the electronic device 104 and the sleep monitoring server 106) connected with the electronic device 2401 through the network. According to one embodiment, the communication module 2420 may include a cellular module 2421, a WiFi module 2423, BT module 2425, a GPS module 2427, an NFC module 2428, and a Radio Frequency (RF) module 2429.

The cellular module 2421 may provide, for example, a voice call, a video call, a text messaging service, or an Internet service through a communication network (e.g., an LTE, an LTE-A, a CDMA, a WCDMA, a UMTS, a WiBro, or a GSM). In addition, the cellular module 2421 may perform discrimination and authentication within the communication network using, for example, a subscriber identification module (e.g., the SIM card 2424). According to one embodiment, the cellular module 2421 performs at least some of the functions which may be provided by the AP 2410. For example, the cellular module 2421 may perform at least some of multimedia control functions.

According to one embodiment, the cellular module 2421 may include a Communication Processor (CP). In addition, the cellular module 2421 may be implemented by an SoC. FIG. 24 illustrates components, such as the cellular module 2421 (e.g., a communication processor), the memory 2430, and the power management module 2495, as separate components from the AP 2410. According to one embodiment, however, the AP 2410 may be implemented to include at least some of the above-mentioned components (*e.g.*, cellular module 2421).

According to one embodiment, the AP 2410 or the cellular module 2421 *(e.g.,* the communication processor) may process a command or data received from a non-volatile memory connected thereto or at least one of the other components by loading the command or data on a volatile memory to be processed. In addition, the AP 2410 or the cellular module 2421 may store data received from at least one of the other components or generated by at least one of the other components in the non-volatile memory.

Each of the WiFi module 2423, the BT module 2425, the GPS module 2427 and the NFC module 2428 may include a processor for processing transmitted/received data. Although FIG. 24 illustrates the cellular module 2421, the WiFi module 2423, the BT module 2425, the GPS module 2427, and the NFC module 2428 as separate components, according to one embodiment, at least some (e.g., two or more) of the cellular module 2421, the WiFi module 2423, the BT module 2425, GPS module 2427, and the NFC module 2428 may be incorporated in one Integrated Chip (IC) or IC package. For example, at least some of the processors, which correspond to the cellular module 2421, the WiFi module 2423, the BT module 2425, the GPS module 2427, and the NFC module 2428, respectively (e.g., a communication processor corresponding to the cellular module 2421 and a WiFi processor corresponding to the WiFi module 2423), may be implemented with one SoC.

The RF module 2429 performs data transmission/reception, for example, transmission/reception of an RF signal. Although not illustrated, the RF module 2429 may include, for example, a transceiver, a Power Amp Module (PAM), a frequency filter, or a low noise amplifier (LNA). In addition, the RF module 2429 may further include a part for transmitting/receiving electromagnetic waves in a free space in a wireless communication, for example, a conductor or a conducting wire. FIG. 24 illustrates that the cellular module 2421, the WiFi module 2423, the BT module 2425, the GPS module 2427, and the NFC module 2428 share one RF module 2429. According to one embodiment, however, at least one of the cellular module 2421, the WiFi module 2423, the BT module 2425, the GPS module 2427, and the NFC module 2428 may perform transmission/reception of an RF signal through one or more separate RF modules.

According to one embodiment, the electronic device 2401 may be connected with a companion device (*e.g*., the electronic device 104) through the WiFi module 2423, the BT module 2425, or the NFC module 2428. In addition, the electronic device 2401 may be connected to a base station *(e.g.,* the server 106) through the cellular module 2421, and connected with a host device *(e.g.,* the electronic device 101) through the base station.

The SIM card 2424 may be a card including a subscriber identification module, and may be inserted into a slot formed at a specific position in the electronic device. The SIM card 2424 may include unique identification information, such as, *e.g.,* Integrated Circuit Card Identifier (ICCID) or subscriber information (*e.g*., International Mobile Subscriber Identity (IMSI)).

The memory 2430 may include an embedded memory 2432 and/or an external memory 2434. The embedded memory 2432 may include at least one of, for example, a volatile memory (*e.g*., a Dynamic RAM (DRAM), a Static RAM (SRAM), or a Synchronous Dynamic RAM (SDRAM)), and a non-volatile memory *(e.g.,* a One Time Programmable ROM (OTPROM), a Programmable ROM (PROM), an Erasable and Programmable ROM (EPROM), an Electrically Erasable and Programmable ROM (EEPROM), a mask ROM, a flash ROM, NAND flash memory, or NOR flash memory).

According to one embodiment, the embedded memory 2432 and/or the external memory 2434 may be a Solid State Drive (SSD). The external memory 2434 may be a flash drive, for example, a Compact Flash (CF), a Secure Digital (SD), a micro-SD, a mini-SD, an extreme digital (xD), or a memory stick. The external memory 2434 may be functionally connected with the electronic device 2401 via various interfaces. According to one embodiment, the electronic device 2401 may further include a storage device (or a storage medium) such as a hard drive.

The sensor module 2440 measures a physical amount or senses an operating state of the electronic device 2401, and converts the measured or sensed information to an electrical signal. The sensor module 2440 may include at least one of, for example, a gesture sensor 2440A, a gyro sensor 2440B, an atmospheric pressure sensor 2440C, a magnetic sensor 2440D, an acceleration sensor 2440E, a grip sensor 2440F, a proximity sensor 2440G, a color sensor 2440H (*e.g.,* an RGB (red, green, blue) sensor), a biometric sensor 2440I, a temperature/humidity sensor 2440J, an illuminance sensor 2440K, and an Ultra-Violet (UV) sensor 2440M. Additionally or alternatively, the sensor module 2440 may include, for example, an E-nose sensor, an ElectroMyoGraphy (EMG) sensor, an ElectroEncephaloGram (EEG) sensor, an ElectroCardioGram (ECG) sensor, an infrared (IR) sensor , an iris sensor, or a fingerprint sensor. In addition, the sensor module 2440 may include, for example, a Heart Rate Variability (HRV) sensor or a Heart Rate Monitor (HRM) sensor. The sensor module 2440 may further include a control circuit that controls one or more sensors included therein.

The input device 2450 may include a touch panel 2452, a (digital) pen sensor 2454, a key input 2456, or an ultrasonic input device 2458. The touch panel 2452 may recognize a touch input by at least one of, for example, a capacitive type, a pressure-sensitive type, an IR type, and an ultrasonic type. In addition, the touch panel 2452 may further include a control circuit. The capacitive type may recognize a physical contact or proximity. The touch panel 2452 may further include a tactile layer. In such a case, the touch panel 2452 may provide a tactile reaction to the user.

The (digital) pen sensor 2454 may be implemented, for example, in a manner identical or similar to a manner of receiving a user's touch input or using a separate recognition sheet. The key input 2456 may be, for example, a physical button, an optical key or a keypad. The ultrasonic input device 2458 is a device that senses sound waves with a microphone *(e.g.,* the microphone 2488) in the electronic device 2401 through an input instrument that generates an ultrasonic signal. According to one embodiment, the electronic device 2401 may receive an user input from an external device (*e.g.,* a computer or a server) connected through the communication module 2420.

The display 2460 may include a panel 2462, a hologram device 2464, or a projector 2466. The panel 2462 may be, for example, a Liquid-Crystal Display (LCD) or an Active-Matrix Organic Light-Emitting Diode (AMOLED). The panel 2462 may be implemented, for example, to be flexible, transparent, or wearable. The panel 2462 may be configured as one module with the touch panel 2452. The hologram device 2464 may show a stereoscopic image in the air using interference of light. The projector 2466 may project light to a screen to display an image. The screen may be positioned, for example, inside or outside the electronic device 2401. According to one embodiment, the display 2460 may further include a control circuit that controls the panel 2462, the hologram device 2464, or the projector 2466.

The interface 2470 may include, for example, a High-Definition Multimedia Interface (HDMI) 2472, a Universal Serial Bus (USB) 2474, an optical interface 2476, or a D-subminiature (D-sub) 2478. The interface 2470 may be incorporated, for example, in the communication interface 160 illustrated in FIG. 2. Additionally or alternatively, the interface 2470 may include, for example, a Mobile High-definition Link (MHL) interface, a Secure Digital (SD) card/Multi-Media Card (MMC) interface, or an Infrared Data Association (IrDA) standard interface.

The audio module 2480 may bi-directionally convert a sound and an electrical signal. At least some components of the audio module 2480 may be included, for example, in the input/output interface 140 illustrated in FIG. 2. The audio module 2480 may process sound information input or output through, for example, a speaker 2482, a receiver 2484, an earphone 2486, or a microphone 2488.

The camera module 2491 is a device capable of photographing a still image and/or a video image. According to one embodiment, the camera module 2491 may include one or more image sensors (*e.g.*, a front sensor and a rear sensor), a lens (not illustrated), an Image Signal Processor (ISP) (not illustrated), or a flash (not illustrated) (e.g., a LED or a xenon lamp).

The power management module 2495 manages the power of the electronic device 2401. Although not illustrated, the power management module 2495 may include, for example, a Power Management Integrated Circuit (PMIC), a charger IC, or a battery or fuel gauge.

The PMIC may be incorporated, for example, in an integrated circuit or an SoC semiconductor. The charging method may be wired and/or wireless. The charger IC may charge a battery and may prevent inflow of an overvoltage or an overcurrent from a charger. According to one embodiment, the charger IC may include a charger IC for at least one of the wired charging method and the wireless charging method. The wireless charging method includes, for example, a magnetic resonance method, a self-induction method, or an electromagnetic wave method, and an additive circuit for wireless charging, for example, an circuit, such as a coil loop, a resonance circuit, or a rectifier circuit, may be added.

A battery gauge may measure, for example, a residual quantity of the battery 2496 or a voltage, current or temperature during charging. The battery 2496 may store or generate electricity, and supply a power to the electronic device 2401 using the stored or generated electricity. The battery 2496 may include, for example, a rechargeable battery or a solar battery.

The indicator 2497 displays a specific state of the electronic device 2401 or a part thereof (e.g., the AP 2410), for example, a booting state, a message state, or a charging state. The motor 2498 may convert an electrical signal to a mechanical vibration. Although not illustrated, the electronic device 2401 may include a processing device for supporting a mobile TV (e.g., a GPU). The processing for supporting the mobile TV may process media data according to a standard of, for example, a Digital Multimedia Broadcasting (DMB), a Digital Video Broadcasting (DVB), or a media flow.

The aforementioned elements of the electronic device according to various embodiments of the present disclosure may be constituted by one or more components, and the name of the corresponding element may vary with a type of electronic device. The electronic device according to various embodiments of the present disclosure may include at least one of the aforementioned elements. Some elements may be omitted or other additional elements may be further included in the electronic device, as would be understood by one of ordinary skill in the art. In addition, a single entity constituted by combining some elements of the electronic device according to the various embodiments of the present disclosure may equivalently perform functions of multiple elements described above.

The "module" used in various embodiments of the present disclosure may refer to, for example, a "unit" including one of hardware, software, and firmware, or a combination of two or more of the hardware, software, and firmware. The "module" may be interchangeable with a term, such as a unit, a logic, a logical block, a component, or a circuit. The module may be a minimum unit of an integrated component element or a part thereof. The "module" may be the smallest unit that performs one or more functions or a part thereof. The module may be mechanically or electronically implemented. For example, the "module" according to various embodiments of the present disclosure may include at least one of an Application-Specific Integrated Circuit (ASIC) chip, a Field-Programmable Gate Arrays (FPGAs), and a programmable-logic device for performing operations which have been known or are to be developed hereafter.

According to various embodiments, at least a part of the device (e.g., modules or functions thereof) and/or the method (e.g., operations) according to various embodiments of the present disclosure may, for example, be implemented by an instruction stored in a non-transitory computer readable storage medium in the form of a programming module. The instruction, when executed by one or more processors (e.g., the processor 120), may cause the one or more processors to perform a function corresponding to the instruction. The computer-readable storage medium may, for example, be the memory 130 in FIG. 2. At least a part of the programming module may, for example, be implemented (e.g., executed) by the processor 120 in FIG. 2. At least a part of the programming module may, for example, include a module, a program, a routine, a set of instructions, or a process for performing at least one function.

The computer readable recording medium may include magnetic media such as a hard disc, a floppy disc, and a magnetic tape, optical media such as a compact disc read only memory (CD-ROM) and a digital versatile disc (DVD), magneto-optical media such as a floptical disk, and hardware devices specifically configured to store and execute program commands, such as a read only memory (ROM), a random access memory (RAM), and a flash memory. In addition, the program instructions may include high level language codes, which can be executed in a computer by using an interpreter, as well as machine codes made by a compiler. The aforementioned hardware device may be configured to operate as one or more software modules in order to perform the operation of various embodiments of the present disclosure, and vice versa.

A module or a programming module according to the present disclosure may include at least one of the described component elements, a few of the component elements may be omitted, or additional component elements may be included. Operations executed by a module, a programming module, or other component elements according to various embodiments of the present disclosure may be executed sequentially, in parallel, repeatedly (e.g., iteratively), or in a heuristic manner. Further, some operations may be executed in a different order, some of the operations may be omitted, or other operations may be added.

According to various embodiments, there is provided a storage medium that stores commands (or a program). The commands may be those used for executing, in an electronic device, operations of, for example, acquiring a motion sensor value according to a motion of the electronic device; comparing the motion sensor value and a pre-set value for each pre-set section to calculate a motion intensity of the electronic device; determining whether the electronic device is worn by activating a biometric sensor when a change in motion intensity between two or more sections corresponds to a pre-set change; and executing a sleep monitoring operation when the electronic device is worn.

Embodiments of the present invention provided in the present specification and drawings are merely certain examples to readily describe the technology associated with embodiments of the present invention and to help understanding of the embodiments of the present invention, and do not limit the scope of the present invention. Therefore, in addition to the embodiments disclosed herein, the scope of the various embodiments of the present invention should be construed to include all modifications in form or detail covered by the present invention as defined in the appended claims.

## Claims

1. An electronic device (101, 401, 501, 601, 701, 801, 2201, 2204, 2401) comprising:
a motion sensor (210) configured to sense a motion of the electronic device;
a biometric sensor (220) configured to sense a biometric signal; and
a processor (120, 2410) configured to calculate a change in motion intensity of the electronic device by comparing output values of the motion sensor over two or more time periods, to activate the biometric sensor to determine whether the electronic device is currently being worn when the change in motion intensity over two or more time periods corresponds to a predetermined pattern, and to control the electronic device to perform sleep monitoring when it is determined that the electronic device is currently being worn.
when it is determined that the electronic device is currently being worn.

2. The electronic device of claim 1, wherein, when it is determined that the electronic device (101, 401, 501, 601, 701, 801, 2201, 2204, 2401) is not currently being worn, the processor (120, 2410) executes a power saving mode.

3. The electronic device of claim 1, wherein the biometric sensor (220) comprises a photo sensor (221) comprising a light emission unit and a light reception unit, the photo sensor (221) measuring an amount of light received through the light reception unit when light is output through the light emission unit or wherein the biometric sensor (220) comprises a Galvanic Skin Response (GSR) sensor (223, 2440F) that measures an electrical conductivity between two predetermined contact points, or wherein the biometric sensor (220) comprises a temperature sensor (225, 2440J) that measures a temperature.

4. The electronic device of claim 1, wherein the predetermined pattern is when the motion intensity changes from a time period in which the motion intensity is larger than a predetermined threshold value to a next time period in which the motion intensity is smaller than the predetermined threshold value.

5. The electronic device of claim 1, wherein the processor (120, 2410) calculates the change in motion intensity of the electronic device (101, 401, 501, 601, 701, 801, 2201, 2204, 2401) over a predetermined time period and the predetermined time period comprises at least one of 1 minute, 30 seconds, and 2 minutes.

6. The electronic device of claim 1, wherein, when the change in motion intensity between two or more time periods does not correspond to the predetermined pattern, the processor (120, 2410) maintains a previous state.

7. A method for sleep monitoring in an electronic device, comprising:
acquiring motion sensor values according to a current motion of the electronic device;
calculating a change in motion intensity of the electronic device by comparing the motion sensor values over two or more time periods;
determining whether the change in motion intensity over two or more time periods corresponds to a predetermined pattern;
if it is determined that the change in motion intensity corresponds to the predetermined pattern, determining whether the electronic device is currently being worn; and
if it is determined that the electronic device is currently being worn, performing sleep monitoring.

8. The method of claim 7, further comprising:
if it is determined that the electronic device is not currently being worn, entering a power saving mode.

9. The method of claim 7, wherein determining whether the electronic device is currently being worn comprises:
determining whether the electronic device is currently being worn using a measured amount of light received through a light reception unit of a photo sensor when light is output through a light emission unit of the photo sensor,
determining whether the electronic device is currently being worn using the calculated resistance value by calculating a resistance value using a measured electrical conductivity between two predetermined contact points through a Galvanic Skin Response (GSR) sensor, or
determining whether the electronic device is currently being worn using a measured temperature.by measuring a temperature using a temperature sensor.

10. The method of claim 7, wherein the predetermined pattern is when the motion intensity changes from a time period in which the motion intensity is larger than a predetermined threshold value to a next time period in which the motion intensity is smaller than the predetermined threshold value.

11. The method of claim 7, wherein the change in motion intensity is calculated over a predetermined time period, and the predetermined time period is comprises at least one of 1 minute, 30 seconds, and 2 minutes.

12. The method of claim 7, further comprising:
if it is determined that the change in motion intensity does not correspond to the predetermined pattern, maintaining a previous state of the electronic device.

13. A method for sleep monitoring in an electronic device, comprising:
acquiring a motion sensor value according to a current motion of the electronic device;
transmitting the acquired motion sensor value to an external electronic device;
if a sensor module activation request is received from the external electronic device, activating a sensor module;
acquiring a sensor value using the activated sensor module;
transmitting the sensor value to the external electronic device for determining whether the electronic device is currently being worn;
receiving a sleep monitoring mode request from the external electronic device when the external electronic device determines, based on the transmitted sensor value, that the electronic device is currently being worn; and
performing sleep monitoring according to the received sleep monitoring mode request by detecting and transmitting sensor values to the external electronic device.

14. A method for sleep monitoring in an electronic device, comprising:
receiving motion sensor values from the electronic device, the motion sensor values detected according to a current motion of the electronic device;
calculating a change in motion intensity of the electronic device by comparing the motion sensor values over two or more time periods and determining whether the change in motion intensity over two or more time periods corresponds to a predetermined pattern;
if it is determined that the change in motion intensity between two or more periods corresponds to the predetermined pattern, transmitting a sensor module activation request to the electronic device;
receiving a sensor value from the electronic device;
determining whether the electronic device is currently being worn using the received sensor value;
if it is determined that the electronic device is currently being worn, transmitting a sleep monitoring mode request to the electronic device; and
performing sleep monitoring by using sensor values received from the electronic device while it is in sleep monitoring mode.
